# FASCICULE DE BREVET EUROPEEN

(11) **EP 2 951 591 B1**
(45) Date de publication et mention de la délivrance du brevet: **17.04.2019**
(21) Numéro de dépôt: 14708597.1
(22) Date de dépôt: 31.01.2014
(51) Int. Cl.: G01N 33/574

(54) **PROCEDE DE DETECTION D'UNE LESION COLORECTALE**
VERFAHREN ZUR ERKENNUNG EINES KOLOREKTALEN LÄSIONEN
METHOD FOR DETECTING A COLORECTAL LESION

(30) Priorité: 01.02.2013 FR 1350908
(43) Date de publication de la demande: 09.12.2015
(73) Titulaire: Biomérieux, 69280 Marcy L'Etoile (FR); CHU DE DIJON, 21079 Dijon Cedex (FR)
(72) Inventeur: CHARRIER, Jean-Philippe, 69160 Tassin La Demi-Lune (FR); CHOQUET-KASTYLEVSKY, Geneviève, 69340 Francheville (FR); FORTIN, Tanguy, 69006 Lyon (FR); HAIDOUS, Hader, 42000 Saint Etienne (FR); LEMOINE, Jérôme, 69480 Lucenay (FR); SALVADOR, Arnaud, 69100 Villeurbanne (FR); FAIVRE, Jean, 21240 Talant (FR)
(74) Mandataire: Agasse, Stéphane
(86) Numéro de dépôt international: PCT/FR2014/050177
(87) Numéro de publication internationale: WO 2014/118475

(56) Documents cités:
- WO-A1-00/33083
- WO-A2-2009/019368
- FRANCIS F. LAM ET AL: "Identification of distinctive protein expression patterns in colorectal adenoma", PROTEOMICS - CLINICAL APPLICATIONS, vol. 4, no. 1, 1 janvier 2010 (2010-01-01) , pages 60-70, XP055019699, ISSN: 1862-8346, DOI: 10.1002/prca.200900084
- PEI HAIPING ET AL: "Proteome analysis and tissue microarray for profiling protein markers associated with lymph node metastasis in colorectal cancer", JOURNAL OF PROTEOME RESEARCH, ACS, WASHINGTON, DC, US, vol. 6, no. 7, 6 juillet 2007 (2007-07-06) , pages 2495-2501, XP002596851, ISSN: 1535-3893, DOI: 10.1021/PR060644R [extrait le 2007-06-02]
- LYALL MATTHEW S ET AL: "Profiling markers of prognosis in colorectal cancer", CLINICAL CANCER RESEARCH, THE AMERICAN ASSOCIATION FOR CANCER RESEARCH, US, vol. 12, no. 4, 15 février 2006 (2006-02-15), pages 1184-1191, XP002432253, ISSN: 1078-0432, DOI: 10.1158/1078-0432.CCR-05-1864
- LAWRIE L C ET AL: "Liver fatty acid binding protein expression in colorectal neoplasia", BRITISH JOURNAL OF CANCER, NATURE PUBLISHING GROUP, GB, vol. 90, no. 10, 17 mai 2004 (2004-05-17), pages 1955-1960, XP002462481, ISSN: 0007-0920, DOI: 10.1038/SJ.BJC.6601828

### SEQUENCE LISTING

<110> Biomérieux Centre Hospitalier Universitaire de Dijon
<120> Procédé de détection d'une lésion colorectale
<130> BR077487
<160> 7
<170> PatentIn version 3.5
<210> 1
   <211> 56
   <212> PRT
   <213> Homo sapiens
<400> 1
   Met Ser Phe Ser Gly Lys Tyr Gln Leu Gln Ser Gln Glu Asn Phe Glu
   1               5                   10                  15
   Ala Phe Met Lys Ala Ile Gly Leu Pro Glu Glu Leu Ile Gln Lys Gly
               20                  25                  30
   Lys Asp Ile Lys Gly Val Ser Glu Ile Val Gln Asn Gly Lys His Phe
           35                  40                  45
   Lys Phe Thr Ile Thr Ala Gly Ser
       50                  55
<210> 2
   <211> 65
   <212> PRT
   <213> Homo sapiens
<400> 2
   Met Ser Phe Ser Gly Lys Tyr Gln Leu Gln Ser Gln Glu Asn Phe Glu
   1               5                   10                  15
   Ala Phe Met Lys Ala Ile Gly Leu Pro Glu Glu Leu Ile Gln Lys Gly
               20                  25                  30
   Lys Asp Ile Lys Gly Val Ser Glu Ile Val Gln Asn Gly Lys His Phe
           35                  40                  45
   Lys Phe Thr Ile Thr Ala Gly Ser Lys Val Ile Gln Asn Glu Phe Thr
       50                  55                  60
   Val
   65
<210> 3
   <211> 12
   <212> PRT
   <213> Homo sapiens
<400> 3
   His Leu Ser Glu Tyr His Pro Trp His Pro Arg Ala
   1               5                   10
<210> 4
   <211> 26
   <212> PRT
   <213> Homo sapiens
<400> 4
   Thr Glu Leu Asn Gly Asp Ile Ile Thr Asn Thr Met Thr Leu Gly Asp
   1               5                   10                  15
   Ile Val Phe Lys Arg Ile Ser Lys Arg Ile
               20                  25
<210> 5
   <211> 11
   <212> PRT
   <213> Homo sapiens
<400> 5
   Ala Ile Gly Leu Pro Glu Glu Leu Ile Gln Lys
   1               5                   10
<210> 6
   <211> 10
   <212> PRT
   <213> Homo sapiens
<400> 6
   Gly Val Ser Glu Ile Val Gln Asn Gly Lys
   1               5                   10
<210> 7
   <211> 10
   <212> PRT
   <213> Homo sapiens
<400> 7
   Thr Val Val Gln Leu Glu Gly Asp Asn Lys
   1               5                   10

## Description

L'invention concerne la détection de lésions colorectales à un stade non cancéreux mais susceptibles d'évoluer en cancer colorectal invasif, et la détection de tumeurs in situ (Tis, aussi appelées carcinomes in situ ou intra-muqueux, de stade TO), également susceptibles de dégénérer en cancers invasifs.

Du fait de leurs similitudes, les cancers du côlon et du rectum sont généralement regroupés sous le terme de cancer colorectal (CCR). Les tumeurs du cancer colorectal se développent aux dépens de la muqueuse du côlon (ou colique), le plus souvent au niveau du sigmoïde, qui est situé dans la dernière partie du côlon, en ce qui concerne les cancers du côlon, et en-dessous de cette zone, en ce qui concerne les cancers du rectum. La forme la plus commune de ces cancers est appelée adénocarcinome.

La muqueuse normale colique est constituée d'un épithélium glandulaire monostratifié, polarisé, continu, organisé sous forme de cryptes, limité par une membrane basale, et entouré par une lamina propria. Les cryptes sont disposées perpendiculairement à la musculaire muqueuse. La lamina propria, autrement appelée chorion, a un contenu cellulaire (cellules mésenchymateuses et inflammatoires), stromal et vasculaire, variable en fonction de la localisation dans le colon. Sa limitation en profondeur est constituée par la musculaire muqueuse. Dans la lamina propria, la vascularisation est limitée à des capillaires et à des veinules endothéliales, et à des canaux lymphatiques superficiellement à la musculaire muqueuse.

Il existe différents types de tumeurs bénignes, c'est-à-dire non cancéreuses, colorectales. Le terme macroscopique de polype désigne toute masse sessile ou pédiculée faisant protrusion dans la lumière colique, quelle qu'en soit sa nature histologique. Sur le plan histologique, les adénomes sont susceptibles de se transformer en cancer. Les polypes hyperplasiques ne sont pas des lésions précancéreuses. Il en est de même des pseudo-polypes inflammatoires ou des polypes juvéniles. La carcinogénèse colique est relativement bien connue. En occident, le cancer colorectal se développe dans 60 à 85% des cas à partir d'un adénome. Celui-ci peut être pédiculé, sessile ou même être à peine en relief dans le cas de l'adénome plan. Un adénome est un foyer circonscrit de dysplasie épithéliale. Il est généralement admis que sur 1 000 adénomes, 100 atteindront la taille de 1 cm et 25 deviendront des cancers dans un délai de 10 à 20 ans. La probabilité cumulative de transformation cancéreuse d'un adénome de diamètre supérieur ou égal à 1 cm a été calculée. Elle est de 2,5 % à 5 ans, 8 % à 10 ans et 24 % à 20 ans. Le risque de transformation carcinomateuse croît avec le nombre, la taille de l'adénome (>=1 cm), la présence d'un contingent villeux et l'existence d'une dysplasie sévère. Les adénomes présentant au moins un de ces critères constituent les adénomes à risque élevé de transformation colique.

La séquence adénome-carcinome est à présent bien établie :
- la prévalence des adénomes à risque élevé de transformation maligne et des carcinomes invasifs colorectaux est élevée dans les mêmes populations ;
- le pic d'incidence des polypes adénomateux précède de quelques années celui des cancers ;
- le risque de cancer est fonction du nombre des adénomes, ce qui explique le caractère inéluctable de transformation des polyposes familiales qui sont caractérisées par la présence de plus de 200 adénomes ;
- on observe fréquemment sur les pièces de colectomie des lésions adénomateuses au contact du foyer carcinomateux infiltrant ;
- l'incidence du cancer colorectal est diminuée après exérèse des adénomes.

On évalue le degré de dysplasie des adénomes en fonction des anomalies d'architecture, de cytologie et de différenciation. Tous les adénomes sont dysplasiques, mais on distingue les dysplasies de bas grade (anciennement légères ou modérées) et de haut grade (sévères). Tout adénome bénin est par définition en dysplasie de bas grade, mais peut par la suite évoluer en adénome en dysplasie de haut grade, généralement associée à son augmentation de taille de plus de 1 cm de diamètre.

En pratique, la séquence adénome-cancer progresse depuis l'adénome en dysplasie de bas grade jusqu'au carcinome invasif, avec les différentes étapes ci-dessous :
- Dysplasie de bas grade
- Dysplasie de haut grade
- Tumeur in situ (Tis, ou carcinome intra épithélial ou intra muqueux)
- Carcinome invasif à partir de l'envahissement de la sous-muqueuse.

De façon générale, le cancer in situ est un cancer au stade initial de son développement, restant limité au tissu qui lui a donné naissance. Sa définition est microscopique. Dans les carcinomes invasifs du côlon, les cellules malignes ont franchi la musculaire muqueuse et atteignent au moins la sous-muqueuse (stade T1 de la classification TNM) ou la musculeuse (stade T2). La musculaire muqueuse est une fine couche de muscles lisses, traversée par des complexes lymphoglandulaires, des canaux vasculaires et des cellules nerveuses.

Lorsque l'on progresse du stade TO au stade T4, il existe à l'échelon tissulaire une désorganisation architecturale de plus en plus importante. L'extension se fait vers la séreuse, avec souvent une invasion des lymphatiques. L'infiltration du cancer peut être associée à une invasion vasculaire ou à un engainement péri-nerveux par les cellules tumorales. Le carcinome peut être plus ou moins bien différencié, quel que soit son stade.

C'est la dégradation de la membrane basale puis le franchissement de la musculaire muqueuse qui permettront la migration des cellules tumorales et la diffusion du cancer. Les carcinomes colorectaux invasifs sont classés suivant la classification internationale TNM en cancers de stade I à IV.

Les tumeurs in situ TO (Tis, ou carcinomes intra-épithéliaux ou intra-muqueux) pourraient être considérés comme correspondant à un stade 0, mais qui n'existe pas dans la classification TNM, car ils ne sont pas considérés comme des cancers, n'étant pas invasifs, et pas susceptibles d'être accompagnés d'envahissement ganglionnaire ni de métastases. Par contre, ce sont des lésions susceptibles d'évoluer en cancer invasif et qu'il faut donc détecter pour en faire l'exérèse et prévenir l'évolution vers un cancer invasif.

En pratique, la sécrétion des marqueurs tumoraux est en général proportionnelle au stade des tumeurs. La désorganisation architecturale, l'envahissement tissulaire, et la présence d'emboles vasculaires favorisent le relargage de marqueurs tumoraux dans les fluides biologiques, comme ils favorisent la dissémination du cancer à distance par la migration des cellules tumorales. Lorsque la membrane basale n'est pas franchie, comme c'est le cas dans les adénomes et dans les carcinomes non invasifs intra-épithéliaux, ou lorsque le cancer est strictement intra-muqueux, le relargage de marqueurs dans la circulation est très improbable, ce qui n'est pas le cas lorsque la tumeur est invasive et en particulier lorsqu'il y a des emboles vasculaires ou nerveux. De façon inattendue, la Demanderesse a découvert que la protéine Liver Fatty Acid-Binding Protein (LFABP) constitue un marqueur, à distance, de lésions colorectales non cancéreuses et de tumeurs in situ. La détection de telles lésions dont l'évolution en carcinome invasif est significativement élevée, permet leur exérèse, et donc de réduire le nombre de cancer colorectal invasif et la mortalité due à ce cancer.

Cette protéine, LFABP (N° Swiss Prot P07148, également appelée L-FABP, FABP1, FABPL, protéine Z ou protéine transporteur de stérol) appartient à la famille des FABP qui comprend neuf isoformes. Chaque isoforme est dénommée d'après le tissu dans lequel elle a été détectée la première fois. Ces isoformes possèdent une communauté de fonctions, des structures tridimensionnelles ressemblantes mais leur homologie de séquence n'est pas élevée. La LFABP a été séquencée en 1985. C'est une petite protéine de 15 kDa, abondante dans le cytosol, possédant la capacité de se fixer aux acides gras libres ainsi qu'à la bilirubine.

Pour le cancer du côlon, plusieurs équipes ont identifié une diminution de l'expression de la protéine LFABP au niveau du tissu tumoral (néoplasique ou pré-néoplasique) comparée à la muqueuse colique normale, en utilisant des techniques d'électrophorèse en 2 dimensions. Ce résultat a aussi été confirmé par des techniques d'immunohistochimie. L'expression tissulaire de la LFABP est diminuée dans la tumeur primaire colorectale chez des patients porteurs d'atteintes ganglionnaires, comparés à des patients sans atteinte ganglionnaire.

Par ailleurs, l'expression de l'ARNm de la LFABP et la protéine elle-même sont diminuées au cours de la séquence « tissu normal / adénome / cancer colorectal », comme cela a été montré par de nombreux auteurs [Lee et al., Differential expression in normal-adenoma-carcinoma sequence suggests complex molecular carcinogenesis in colon. Oncol Rep. 2006 Oct;16(4):747-54*],* ce qui signifie qu'il y devrait y avoir moins de LFABP dans le tissu des adénomes que dans le tissu normal. Lauriola et al. [Lauriola M et al., Identification by a Digital Gene Expression Displayer (DGED) and test by RT-PCR analysis of new mRNA candidate markers for colorectal cancer in peripheral blood. Int J Oncol. 2010 Aug;37(2):519-25] ont essayé de quantifier les ARNm circulants dans le sang pour la LFABP et pour d'autres marqueurs, sans arriver à détecter la LFABP chez les patients cancers, ni chez les sujets contrôles.

Dans la demande de brevet WO2009/019368A2, la Demanderesse a mis en évidence que la détection de la protéine LFABP, en combinaison avec les marqueurs ACE (antigene carcino-embryonnaire) et CA 19-9, permettait d'améliorer la détection des patients atteints de cancer colorectal.

La Demanderesse a maintenant découvert de façon surprenante que la LFABP est relarguée par les lésions coliques non cancéreuses, c'est à dire les adénomes et les lésions non invasives Tis TO, et ceci de façon abondante, ce qui n'était pas prévisible puisque dans les lésions coliques non invasives la membrane basale n'est pas franchie rendant plus qu'improbable le relargage de marqueurs dans la circulation. La FABP peut alors être détectée dans les échantillons biologiques distants des tumeurs.

Ainsi, un objet de l'invention est un procédé de détection d'une lésion colorectale non cancéreuse susceptible d'évoluer en carcinome (ou cancer) colorectal invasif, chez un patient n'ayant pas de cancer colorectal invasif, par la détermination de la présence de la Liver Fatty Acid-Binding Protein (LFABP) et par la mise en évidence d'une variation de l'expression de la LFABP par comparaison à une population de référence dite contrôle. Selon le procédé de l'invention, on détecte une telle lésion colorectale, *in vitro*, dans un échantillon biologique du patient choisi parmi le sang total, le sérum et le plasma.

Une application de l'invention est un procédé de dépistage, *in vitro,* d'une lésion colorectale dans une population par détermination de la présence de la LFABP dans un échantillon biologique d'un individu.

Un procédé de l'invention peut en outre comprendre une mise en évidence d'une variation de l'expression de la LFABP. Avantageusement, on met en évidence une augmentation de l'expression de la LFABP. Cette variation peut être observée par comparaison à une population de référence dite contrôle, de patients non suspectés de CCR, par exemple des patients ayant eu une coloscopie qui s'est révélée normale, ou par comparaison à une valeur de référence prédéterminée. De préférence, la variation est observée par rapport à une population de référence, pour des échantillons de même nature.

L'invention concerne aussi les applications d'un procédé de l'invention. Elle concerne notamment un procédé de détection d'une susceptibilité à un cancer colorectal, chez un patient, qui met en oeuvre un procédé de l'invention. Selon une variante, cette susceptibilité est réalisée *in vitro,* par une détection dans un échantillon biologique.

Selon l'invention, on entend par lésion colorectale susceptible d'évoluer en carcinome, ou cancer, colorectal invasif, une lésion non cancéreuse, telle qu'un adénome, un adénome dysplasique avec une dysplasie de bas grade ou une dysplasie de haut grade, un carcinome in situ (intra épithélial ou intra muqueux), tel que défini précédemment. En effet, comme expliqué ci-dessus, un carcinome in situ n'est pas invasif, il est au stade 0 (T0), mais du fait de son histologie, sa probabilité d'évoluer en cancer invasif est plus élevée que celle d'un adénome dysplasique.

Par relarguage par les tumeurs coliques, on entend selon l'invention, la sécrétion active ou passive ou la libération, quel qu'en soit le mécanisme, du marqueur tumoral par les lésions colorectales.

Par rapport aux autres marqueurs tumoraux connus, comme l'antigène carcino-embryonnaire ou le CA 19-9, la détection de la LFABP permet de prévenir un cancer colorectal invasif (CCR).

Par rapport à l'art antérieur, non seulement, ce marqueur permet de prévenir un CCR, en l'absence de toute suspicion de CCR invasif chez le patient, mais il peut être détecté dans un échantillon biologique, et très avantageusement un échantillon distant de la lésion.

Par échantillon biologique, on entend tout échantillon susceptible de contenir la LFAPB et/ou au moins un traceur représentatif de la LFAPB. Cet échantillon peut provenir d'un prélèvement de tout fluide biologique, comme le sang total, le sérum, le plasma, l'urine, le liquide céphalo-rachidien, les sécrétions organiques, la salive, les épanchements, les selles, la moelle osseuse, et les cellules purifiées à partir de ces échantillons liquides, ou d'un prélèvement tissulaire ou un tissu, ou des cellules isolées. De préférence, l'échantillon est distant de la lésion potentielle.

Par détermination de la présence ou de l'expression de la LFABP, on comprend qu'on détecte la LFABP et/ou au moins un traceur représentatif de la LFABP. Ce traceur est caractéristique de la présence de la LFABP. Selon la teneur en LFABP chez le patient ou dans l'échantillon biologique et selon la technique employée, la détection d'un traceur précité peut être plus pertinente que celle de la LFABP en soi. Ainsi, un traceur peut être choisi parmi les précurseurs de la LFABP, les métabolites de la LFABP et toute molécule associée à l'activité biologique de la LFABP, notamment à l'activité enzymatique de la LFABP, des produits de dégradation ou de clivage de la LFABP, ou à une réponse immune due à la LFABP. Ainsi, un traceur de la LFABP est de préférence choisi parmi des peptides, de préférence des mélanges de peptides de la LFABP ; des acides nucléiques, de préférence des ARN messagers ; une modification du gène codant pour la LFABP, comme une méthylation de son promoteur, ou une modification de l'acide nucléique conduisant à une modification de l'expression de la LFABP ; des anticorps reconnaissant spécifiquement la LFABP et produits chez le patient en réponse à une présence de la LFABP, par réaction immunogénique.

Un traceur préféré est un anticorps choisi parmi ceux reconnaissant spécifiquement un peptide de séquence choisie parmi SEQ ID NO : 1, SEQ ID NO : 2, SEQ ID NO : 3 et SEQ ID NO : 4.

D'autres traceurs avantageux sont choisis parmi des peptides présentant les séquences suivantes SEQ ID NO : 1, SEQ ID NO : 2, SEQ ID NO : 3, SEQ ID NO : 4, SEQ ID NO : 5, SEQ ID NO : 6 et SEQ ID NO : 7, et leurs mélanges.

Plus particulièrement, selon un procédé de détection d'une lésion colorectale, on détecte la LFABP et/ou au moins un traceur représentatif de la LFABP et/ou on mesure au moins une activité biologique, par exemple enzymatique, spécifique de la LFABP, dans ledit échantillon biologique.

Les différents procédés décrits ci-dessus peuvent être mis en oeuvre par toute technique appropriée et bien connue de l'homme du métier.

Ainsi, on peut doser la LFABP par une technique quantitative de spectrométrie de masse, par toute technique immunologique, ou une combinaison des deux techniques, ou par toute technique enzymatique. On peut utiliser par exemple la technique LC-MRM-MS qui combine la chromatographie liquide et la spectrométrie de masse en mode MRM (Mutiple Reaction Monitoring).

Le procédé de l'invention peut être amélioré en détectant, outre la LFABP, au moins un autre marqueur permettant de détecter une lésion colorectale à un stade non cancéreux, le cas échéant également relargué par les lésions coliques hors des tissus. Ainsi, la combinaison d'au moins deux marqueurs permet d'améliorer la spécificité et la sensibilité du test de diagnostic des lésions susceptibles d'évoluer en CCR invasif.

Différentes variantes de mise en oeuvre du procédé sont exposées dans les exemples suivants, en faisant référence aux figures 1-5 selon lesquelles :
La Figure 1 représente les résultats du dosage ELISA de la LFABP sur Vidas®, chez des patients contrôles présentant une coloscopie négative (ColoNeg) et des patients présentant des adénomes, tel que décrit à l'exemple 3.
La Figure 2 représente les résultats du dosage ELISA de la LFABP sur Vidas® chez des patients contrôles présentant une coloscopie négative (ColonNeg), et des patients présentant des Tumeurs in situ (Tis T0),, tel que décrit à l'exemple 4.
Les Figures 3, 4 et 5 représentent les résultats du dosage ELISA sur Vidas® de la LFABP, du marqueur ACE et du marqueur CA19-9, respectivement, chez des patients à différents stades de tumeurs, stade Tis et des CCR invasifs de stades TNM I à IV, tel que décrit à l'exemple 4.

### Exemple 1 : Obtention d'anticorps monoclonaux dirigés contre la LFABP

### 1) Obtention de la protéine recombinante

Pour réaliser des immunisations et obtenir des anticorps monoclonaux contre la protéine LFABP, la protéine recombinante LFABP a d'abord été obtenue.

L'ADN complémentaire, les vecteurs d'expression, le clonage et l'obtention de la protéine recombinante LFABP (expression et purification) ont été réalisés de la même façon que décrits dans le brevet WO2010/004214.

### 2) Immunisations

### 2.1) Modèle animal

Les expériences d'immunisation ont été réalisées chez des souris BALB/c (H-2^{d}) femelles âgées de 6 à 8 semaines au moment de la première immunisation.

### 2.2) Immunogènes et immunisations

Afin d'augmenter les réponses immunes obtenues chez les souris et pouvoir générer des anticorps monoclonaux, la protéine LFABP a été produite sous forme de protéine recombinantes produite selon les modes opératoires décrits dans le brevet WO2010/004214A1. La protéine a été mélangée volume pour volume avec l'adjuvant de Freund (Sigma), préparé sous forme d'émulsion eau-dans-huile et dont il est connu qu'il présente un bon pouvoir immunogène. Trois souris ont été immunisées. Les souris ont reçu 3 doses successives de 10 µg des immunogènes à 0, 2 et 4 semaines. Toutes les injections ont été réalisées par voie sous-cutanée. La première injection est faite en mélange avec l'adjuvant de Freund complet, les deux suivantes se font en mélange avec l'adjuvant de Freund incomplet. Entre J50 et J70 après la première injection, les réponses humorales ont été restimulées avec une injection intraveineuse de 100 µg de la protéine recombinante.

### 2.3) Suivi de l'apparition de la réponse humorale

Afin de suivre l'apparition des anticorps, on effectue régulièrement sur les souris des prélèvements de sang. La présence des anticorps anti-marqueur tumoral est testée en utilisant un ELISA. La protéine d'intérêt est utilisée en capture (1 µg/puits), après saturation on fait réagir avec l'antigène différentes dilutions des sérums à tester (incubation à 37°C, pendant 1h). Les anticorps spécifiques présents dans le sérum sont révélés par un anticorps de chèvre anti-IgG de souris AffiniPure conjugué à la phosphatase alcaline (H+L, Jackson Immunoresearch, Cat no. 115-055-146), qui se lie aux anticorps recherchés (0,1 µg/puits).

### 2.4) Obtention d'anticorps monoclonaux

Trois jours après la dernière injection, pour chaque marqueur tumoral, une des souris immunisées a été sacrifiée ; le sang et la rate ont été prélevés. Les splénocytes obtenues à partir de la rate ont été mises en culture avec les cellules de myélome Sp2/0-Ag14 pour qu'elles fusionnent et s'immortalisent, selon le protocole décrit par Köhler et Milstein [G. Köhler et C. Milstein, 1975, Nature, 256, 495-497 ; G. Köhler et C. Milstein, 1976, Eur J Immunol, 6, 511-519].

Après une période d'incubation de 12-14 jours les surnageants des hybridomes obtenus ont été criblés pour déterminer la présence d'anticorps anti-marqueur tumoral en utilisant le test ELISA décrit dans le point 3 de cet exemple. Les colonies d'hybridome positives ont été sous-clonées deux fois selon la technique de la dilution limite, bien connue par l'homme du métier.

### 3) Caractérisation des anticorps monoclonaux

La liste des anticorps monoclonaux obtenus est présentée dans le Tableau 1. Ces anticorps monoclonaux ont été analysés par la technique du Western blot.

**Tableau 1**

| Anticorps monoclonaux |
|---|
| 2E5G9 |
| 3A7B4 |
| 5A8H2 |
| 2C9G6 |
| 3D6G1 |
| 8B10B8 |
| 7D8A11 |

### 3.1) Complémentarité des anticorps

Pour pouvoir construire le test ELISA permettant de doser les échantillons de patients, on a tout d'abord testé la complémentarité des anticorps. Ce sont les mêmes anticorps qui sont utilisés en capture ou en révélation (après biotinylation). Chaque anticorps de révélation biotinylé est testé avec chaque anticorps de capture (anticorps du cône VIDAS®). Les couples sont testés avec la protéine recombinante LFABP décrite ci-dessus. Le tableau 2 récapitule les résultats obtenus pour les différents couples d'anticorps.

**Tableau 2**

| Tableau croisé montrant les résultats obtenus pour chaque couple d'anticorps avec la protéine recombinante LFABP | | | | | | | |
|---|---|---|---|---|---|---|---|
| (moyenne du signal - moyenne du bruit de fond en RFV). (RFV ou Relative Fluorescence Value est le signal lu par Vidas®®). | | | | | | | |
| | Détection | | | | | | |
| Capture | 2E5G9 | 3A7B4 | 5A8H2 | 2C9G6 | 3D6G1 | 8B10B8 | 7D8A11 |
| 2E5G9 | 10,5 | 16,5 | 25 | 10537 | 10377,5 | 5066 | 0,5 |
| 3A7B4 | 42 | 198 | 707,5 | 10071 | 9923 | 10009 | 24,5 |
| 5A8H2 | 36 | 109 | 513 | 10404 | 10283,5 | 9930 | 18,5 |
| 2C9G6 | 10753,5 | 10815 | 10708,5 | 1417,5 | 213 | 10774,5 | 1562 |
| 3D6G1 | 10136,5 | 10707,5 | 10684,5 | 606,5 | 138 | 10462 | 1038,5 |
| 8B10B8 | 8846 | 9940,5 | 9677 | 10385,5 | 10637,5 | 87 | 363,5 |
| 7D8A11 | 5,5 | 21,5 | 14 | 8053,5 | 8094,5 | 128 | 4,5 |

A l'issue de ces tests, on retient les 6 couples d'anticorps complémentaires suivants :
- 3A7B4/3D6G1
- 5A8H2/3D6G1
- 2C9G6/5A8H2
- 3D6G1/5A8H2
- 8B10B8/5A8H2
- 2C9G6/2E5G9

### 3.2) Caractérisation des épitopes

Les épitopes reconnus par les anticorps monoclonaux ont été caractérisés en utilisant les techniques du Spotscan, le criblage de banque de peptides portés par des phages, et la construction de protéines chimères (librairie de fragments).

### 3.2.1) Méthodologie

La technique du Spotscan, adaptée d'après Frank et Döring, permet de synthétiser de façon simultanée un grand nombre de peptides fixés sur membrane de cellulose. Ces peptides reproduisent la séquence de l'antigène cible sous forme de peptides de 8 à 12 acides aminés, se chevauchant de 1 à 4 résidus. Ces peptides sont ensuite mis en contact avec l'anticorps à étudier dans un test colorimétrique de type Blot, et l'identification des peptides immunoréactifs permet de déduire la séquence minimale de l'épitope de l'anticorps et de le localiser précisément sur l'antigène.

La synthèse s'effectue sur une membrane de cellulose portant uniformément des bras en polyéthylène glycol (PEG) d'une longueur de 8 à 10 unités, présentant une fonction NH₂ libre en bout de chaîne. Elle se déroule de l'extrémité C-terminale vers l'extrémité N-terminale des peptides. Les acides aminés ont leur fonction aminée protégée par un groupement Fmoc (9-fluoréméthyloxycarbonyl), et leurs chaînes latérales, susceptibles de réagir au cours de la synthèse, sont également protégées par des groupements trityl, t-butyl ou t-butyl-éther. Les solutions-mères d'acides aminés sont préparées à une concentration de 0,33 M dans du NMP (N-méthyl-pyrrolidone) contenant 0,5 M de HOBt (hydroxybenzotriazole). Le dépôt des acides aminés s'effectue à l'aide du robot ASP 222 (Abimed, Langenfeld, Allemagne), piloté par l'intermédiaire du logiciel AutoSpot XL. L'utilisation de ce robot permet de faire en simultané jusqu'à 4 membranes de 96 spots, soit 384 peptides.

Pour un cycle de couplage d'un acide aminé, le robot dépose 0,7 µl de la solution d'acide aminé activé extemporanément (un volume de solution de diisopropyl-carbodiimide 1,1 M dilué en NMP pour 3 volumes de solution-mère d'acide aminé) sur les membranes. Ce dépôt est répété une seconde fois, puis les membranes sont rincées en DMF (N,N-diméthylformamide). Les groupements NH₂ n'ayant pas réagi sont ensuite été acétylés par 4 à 6 incubations de 10 minutes dans une solution d'anhydride acétique 10 % en DMF, afin d'éviter l'apparition de peptides abortifs ou tronqués. Après 3 lavages de 2 minutes en DMF, les groupements Fmoc protégeant la fonction aminée des acides aminés sont clivés par une incubation de 5 minutes dans une solution de pipéridine 20 % en DMF. Après 4 lavages en DMF, les spots sont colorés à l'aide d'une solution de bleu de bromophénol 1 % en DMF, puis la membrane est rincée 3 fois en méthanol et séchée à l'air libre avant le cycle de couplage suivant.

Ce protocole est répété pour l'ajout de chaque nouvel acide aminé. Après le couplage du dernier acide aminé, les peptides sont acétylés afin de permettre le blocage de tous les groupements NH₂ libres, empêchant ainsi l'ajout d'un autre acide aminé. Puis les chaînes latérales de tous les peptides sont déprotégées par l'incubation des membranes dans un bain acide trifluoroacétique / dichlorométhane / triisobutylsilane (5 : 5 : 0,3) pendant 1 heure. Les membranes sont ensuite rincées 4 fois en dichlorométhane, 3 fois en DMF et 3 fois en méthanol avant d'être séchées à l'air libre et conservées à - 20°C jusqu'à l'immunorévélation.

Pour immunorévéler les spots avec un anticorps monoclonal, les membranes sont d'abord rincées en méthanol, puis lavées en TBS (Tris-HCl 50 mM pH 8,0, NaCl 140 mM, KCl 3 mM) avant d'être incubées sur la nuit à température ambiante dans la solution de saturation (solution concentrée 10X à base de caséine (Western Blocking reagent, Roche) diluée en TBS-Tween 20 0,05% (TBS-T) et contenant 5% de saccharose). Après un lavage de 10 minutes en TBS-T, les membranes sont incubées pendant 1h30 à 37°C avec l'anticorps monoclonal dilué à 20 µg/ml en solution de saturation. Les membranes sont ensuite lavées 3 fois en TBS-T, puis sont incubées avec le conjugué anti-souris couplé à la phosphatase alcaline (Jackson Immunoresearch), dilué au 1/2000^{ème} en solution de saturation. Après 2 lavages de 10 minutes en TBS-T, puis 2 lavages en CBS (acide citrique 10 mM pH 7, NaCl 140 mM, KCl 3 mM), le révélateur, préparé extemporanément (5-bromo, 4-chloro, 3-indoyl, phosphate 600 µM, thiazolyl blue tetrazolium bromide 720 µM, et MgCl₂ 5 mM en CBS), est mis en contact avec la membrane pendant 30 à 45 minutes à l'obscurité. Les peptides immunoréactifs apparaissent en bleu-violet. Après 3 rinçages en eau distillée, les membranes sont scannées puis conservées dans l'eau jusqu'à la régénération.

La régénération permet d'éliminer les anticorps et les conjugués fixés sur les peptides, ce qui permet ainsi de réaliser un nouveau test d'immunoréactivité vis-à-vis d'un autre anticorps. Les membranes subissent une série de lavages de 10 minutes chacun : 1 lavage en eau distillée, 6 lavages en DMF, 3 lavages en tampon de régénération A (urée 8 M, SDS (sodium dodecyl sulfate) 35 mM, β-mercaptoethanol 0,1 %), 3 lavages en tampon de régénération B (eau distillée / éthanol / acide acétique 4 : 5 : 1), puis 2 lavages en méthanol. Les membranes sont ensuite séchées à l'air libre avant d'être stockées à -20°C.

La caractérisation des épitopes par criblage de banques de peptides portés par des phages a été réalisée en utilisant le kit commercial PhD12 Phage Display Peptide Library Kit (Cat. No. E#8110S) de New England Biolabs, en suivant les instructions fournies avec le kit, version 2.7 du protocole datant de novembre 2007.

Une troisième technologie de localisation d'épitope a été utilisée : la construction d'une librairie de fragments de protéine LFABP recombinante, contre laquelle on a testé l'affinité des anticorps d'intérêt

### 3.2.2) Résultats

Le Tableau 3 récapitule les épitopes reconnus par 5 anticorps LFABP dont les épitopes ont été analysés par les 3 techniques mentionnées.

**Tableau 3**

| Anticorps | N° épitope | Séquence de l'épitope^{a} | Type d'épitope | Technique |
|---|---|---|---|---|
| *2C9G6* | 1 | N-term 1-56 (SEQ ID NO : 1) | Conformationnel | librairie de fragments |
| *3D6G1* | 1 | N-term 1-56 (SEQ ID NO : 1) | Conformationnel | librairie de fragments |
| *8B10B8* | 1 | N-term 1-56 (SEQ ID NO : 1) | Conformationnel | librairie de fragments |
| *5A8H2* | 2 | N-term 1-65 (SEQ ID NO : 2) (AA 57 à 65 nécessaires) | Séquence nécessaire pour la liaison | Librairie de fragments |
| | 3 | HLSEYHPWHPRA (SEQ ID NO : 3) | Mimotope Epitope conformationnel | Criblage banque de phage |
| *7D8A11* | 4 | 102-127 SEQ ID NO : 4 | Epitope linéaire | Spotscan et librairie de fragments |

| | | | | |
|---|---|---|---|---|
| ^{a}Séquence en acide aminé de la région de fixation de la LFABP à l'anticorps testé. Les nombres entre parenthèses correspondent à la position de l'épitope sur la séquence en acides aminés de la LFABP, la numérotation commençant à la méthionine initiale. | | | | |

Les épitopes reconnus par les anticorps 3A7B4 et 5A8H2 n'ont pas pu être déterminés par la technique du Spotscan, ce qui indique qu'ils ne sont pas linéaires. Le criblage des banques de peptides portés par des phages a permis de sélectionner 1 mimotope (séquence linéaire mimant un épitope) qui réagit avec l'anticorps 5A8H2. La séquence consensus de ce mimotope a été alignée afin de déterminer une séquence consensus (SEQ ID NO : 3) qui est indiquée dans le tableau 3, qui représente la séquence minimale reconnue par l'anticorps. Il n'a pas été possible de retrouver cette séquence consensus dans la structure primaire (ou séquence peptidique) de la LFABP. Ainsi, cette séquence consensus correspond à des résidus situés à différents endroits sur la structure primaire de la protéine mais qui partagent une proximité dans sa structure tridimensionnelle afin de former un épitope conformationnel. Par ailleurs l'utilisation de la librairie de fragments de protéine recombinante a permis de localiser l'épitope pour les anticorps 2C9G6, 3D6G1 et 8B10B8 dans les 56 premiers acides aminés de la protéine (SEQ ID NO :1). L'anticorps 5A8H2 a besoin quant à lui de 9 acides aminés supplémentaires (AA 1 à 65) pour se fixer, site de liaison à l'opposé du site de liaison de l'anticorps 7D8A11 (SEQ ID NO : 2). L'anticorps 7D8A11 est le seul qui ait un épitope linéaire que l'on a pu caractériser en spotscan (SEQ ID NO : 4).

Les anticorps mentionnés dans le tableau 3 ci-dessus constituent un outil de dosage de la LFABP plus sensible par rapport à d'autres anticorps connus de la LFABP.

### Exemple 2 : développement de tests ELISA sandwich contre la protéine LFABP sur l'automate Vidas®

La protéine LFABP a été dosée à l'aide des anticorps décrit dans l'exemple 1 et d'un immuno-essai de type sandwich en utilisant par exemple l'automate d'ELISA Vidas® (bioMérieux). Ce type de test peut aussi être réalisé en microplaque, de façon automatisée ou manuelle. Dans un premier temps, les différents anticorps disponibles ont été testés pour choisir un couple d'anticorps fonctionnel, c'est à dire, au moins un anticorps capable de capturer la protéine biomarqueur (anticorps de capture) et au moins un anticorps capable de révéler la molécule biomarqueur (anticorps de révélation).

Pour ce faire, le test ELISA a été construit en utilisant les réactifs du kit Vidas® HBs Ag Ultra (bioMérieux, Cat. No. 30315).

Les réactifs ont été utilisés tels que décrits dans la notice correspondante (réf. 11728D-FR-2005/05), avec les modifications suivantes :
- Les cônes ont été sensibilisés avec un des 5 Ac de capture sélectionnés utilisés à une concentration de 10 µg/ml.
- Le contenu du deuxième puits de la cartouche HBS Ag Ultra a été remplacé par 300 µl d'anticorps de révélation, couplé à la biotine, dilués à 1 µg/ml dans le tampon du deuxième puits du kit Vidas® HBs Ag Ultra (tampon avec sérum de chèvre et azoture de sodium à 1 g/l).
- L'échantillon de sérum (50µl) a été dilué directement dans le deuxième puits de la cartouche HBS Ag Ultra.
- La réaction ELISA a été réalisée à l'aide de l'automate Vidas® et du protocole HBS dont l'étape d'incubation de l'échantillon avec les anticorps de capture et de révélation avait été porté à 100 cycles.
- Les résultats ont été obtenus sous forme de valeurs brutes après soustraction du bruit de fond (lecture du substrat avant réaction).

Une courbe étalon a été établie en dosant une gamme de concentration de la protéine biomarqueur recombinante. La courbe étalon a été tracée en reportant en abscisse la concentration de la protéine biomarqueur et en ordonnée le signal lu par Vidas® (RFV ou Relative Fluorescence Value). La concentration de la protéine biomarqueur présente dans le fluide corporel à doser (sang, sérum, plasma, selle) a été calculée en reportant la concentration correspondant au signal RFV lu par Vidas®.

### Equivalence des différents tests ELISA développés

Nous avons voulu évaluer la corrélation entre les différents immuno-essais sandwich développés. Les tests Vidas® ont été comparés entre eux et également comparé au test commercialisé par la société Hycult biotechnology pour doser la protéine LFABP humaine (Cat. No. HK404), tel que décrit dans la demande de brevet WO2009019368A2.

Ces tests d'équivalence ont été réalisés grâce à des échantillons sériques de patients atteints de cancer colorectal (CCR) ou de sujet sain contrôles donneurs de sang (EFS), obtenus respectivement dans des services hospitaliers dans le cadre de 2 protocoles loi Huriet, et dans un établissement français de banque du sang (sujets sains).

**Tableau 4**

| Doses de LFABP en ng/ml obtenus avec le test commercial Hycult biotechnology et avec les différents tests Vidas® développés | | | | | | | |
|---|---|---|---|---|---|---|---|
| Patient | Hycult | 2C9G6/5A8H2 | 5A8H2/3D6G1 | 2C9G6/2E5G9 | 3A7B4/3D6G1 | 3D6G1/5A8H2 | 8B10B8/5A8H2 |
| CBSE004 | 7,78 | | 2,87 | 1,74 | 2,57 | | |
| CBSE011 | 7,73 | | 1,92 | 1,33 | 1,72 | | |
| CBSE012 | 0,11 | | 2,31 | 1,83 | 2,07 | | |
| CBSE018 | | | 1,35 | 0,75 | 1,19 | | |
| CBSE019 | 6,65 | | 4,48 | 3,41 | 3,98 | | |
| CBSE023 | 8,21 | | 4,60 | 4,92 | 4,07 | | |
| CBSE025 | 16,38 | | 7,88 | 7,45 | 6,87 | | |
| CLSP043 | 2,89 | 1,94 | 2,49 | 1,70 | 2,47 | 2,29 | 0,22 |
| CLSP047 | 1,94 | | 1,39 | 0,00 | 1,22 | | |
| CLSP078 | 2,56 | | 13,01 | 8,27 | 11,33 | | |
| CLSP090 | 3,77 | | 1,18 | 0,98 | 1,04 | | |
| CLSP164 | 6,54 | 4,18 | 4,77 | 4,60 | 4,49 | 4,14 | 1,54 |
| CLSP165 | 7,86 | 1,27 | 1,68 | 1,80 | 2,32 | 1,82 | 1,64 |
| CLSP167 | 8,17 | 0,00 | 0,43 | 0,00 | 2,01 | 0,00 | 0,52 |
| CLSP170 | 6,02 | 4,36 | 4,26 | 4,61 | 4,16 | 3,60 | 2,11 |
| CLSP174 | 23,64 | 5,57 | 6,73 | 6,26 | 7,80 | 4,67 | 7,39 |
| CLSP182 | 7,25 | 4,47 | 4,61 | 5,50 | 4,46 | 4,21 | 4,06 |
| CLSP183 | 16,90 | 11,07 | 10,12 | 9,79 | 9,32 | 7,08 | 15,94 |
| CLSP185 | 19,63 | 5,35 | 7,01 | 6,36 | 8,41 | 4,44 | 5,32 |
| CLSP186 | 7,06 | 4,59 | 4,42 | 5,35 | 4,44 | 3,39 | 2,72 |
| CLSP189 | 5,34 | 0,00 | 0,25 | 0,00 | 1,16 | 0,00 | 0,69 |
| CLSP194 | 13,09 | 5,38 | 5,65 | 6,71 | 5,40 | 5,17 | 5,52 |
| CLSP198 | 8,16 | 2,20 | 3,10 | 3,14 | 4,24 | 2,88 | 1,94 |
| CLSP207 | 6,02 | 4,60 | 4,71 | 5,58 | 4,67 | 4,01 | 1,91 |
| GHBD020 | 2,24 | | 5,46 | 3,08 | 4,81 | | |
| GHBD021 | 1,84 | | 1,58 | | 1,41 | | |
| GHBD029 | 2,75 | | 3,42 | 2,74 | 3,05 | | |
| N000533 | 3,46 | 0,59 | 1,18 | 0,96 | 1,51 | 1,21 | 1,85 |
| N002301 | 7,78 | | 5,54 | 5,19 | 4,88 | | |
| N005702 | 2,74 | | 1,04 | 0,79 | 0,91 | | |
| N009944 | 3,49 | 1,41 | 2,06 | 2,66 | 1,90 | 1,98 | 1,69 |
| N011147 | 2,86 | 0,92 | 1,52 | 1,82 | 1,41 | 1,50 | 1,16 |
| N011968 | 2,06 | 1,26 | 1,56 | 1,64 | 1,62 | 1,65 | 0,97 |
| N011984 | 5,29 | 0,87 | 1,32 | 1,21 | 1,80 | 1,45 | 1,19 |
| N014106 | 2,66 | 0,45 | 0,91 | 0,66 | 1,25 | 0,89 | 0,74 |
| N015402 | 4,41 | | 1,34 | 0,90 | 1,18 | | |
| N017234 | 4,04 | 1,90 | 2,49 | 3,33 | 2,41 | 2,61 | 2,48 |
| N017269 | 2,89 | 1,46 | 2,13 | 2,38 | 2,00 | 1,97 | 2,01 |
| N018544 | 2,42 | 0,99 | 1,47 | 2,17 | 1,49 | 1,67 | 1,11 |
| N018552 | 2,28 | 0,00 | 0,15 | 0,00 | 0,52 | 0,00 | 0,47 |
| N020501 | 11,37 | | 2,61 | 2,90 | 2,34 | | |
| N045730 | 2,29 | 0,19 | 0,69 | 0,63 | 0,89 | 0,61 | 0,74 |
| N054582 | 1,84 | | 1,45 | 1,65 | 1,28 | | |
| N057046 | 5,59 | | 0,82 | 0,00 | 0,70 | | |
| N057054 | 21,05 | | 2,69 | 3,25 | 2,40 | | |
| N074598 | 2,53 | | 1,53 | 1,13 | 1,36 | | |
| N286613 | 4,11 | 2,38 | 2,54 | 3,30 | 2,56 | 3,00 | 3,83 |
| N286656 | 7,95 | 1,77 | 2,26 | 2,73 | 3,24 | 2,60 | 4,12 |
| N286680 | 4,58 | 2,75 | 2,86 | 3,30 | 2,85 | 3,39 | 1,45 |
| N318050 | 3,93 | 1,56 | 2,16 | | 2,18 | | 2,05 |
| N318341 | 4,46 | 1,72 | 2,38 | 2,86 | 2,12 | 2,40 | 1,86 |
| N318384 | 1,63 | 0,60 | 1,19 | 1,29 | 1,12 | 1,18 | 0,71 |
| N318421 | 3,86 | 0,69 | 1,15 | 1,45 | 1,47 | 1,14 | 1,56 |
| N329630 | 6,90 | 1,24 | 1,69 | 2,34 | 2,28 | 1,82 | 4,04 |
| N376488 | 4,73 | 0,64 | 1,20 | 1,30 | 1,57 | 1,14 | 1,57 |
| N376912 | 2,55 | 0,27 | 0,84 | 0,94 | 1,15 | 0,80 | 1,07 |
| N40776- | 3,46 | 0,62 | 1,00 | 0,98 | 1,35 | 1,15 | 1,21 |
| N418599 | 2,60 | 1,11 | 1,46 | 1,94 | 1,49 | 1,63 | 2,01 |
| N46043- | 4,18 | 0,67 | 1,34 | 1,18 | 1,72 | 1,03 | 0,90 |
| N461993 | 2,92 | 0,00 | 0,16 | 0,01 | 0,69 | 0,00 | 0,73 |
| N462187 | 3,96 | 0,79 | 1,32 | 1,18 | 1,79 | 1,43 | 1,23 |
| N483535 | 6,42 | 0,00 | 0,30 | 0,00 | 1,28 | 0,00 | 0,83 |
| N491678 | 7,73 | | 3,86 | 3,38 | 3,43 | | |
| N494801 | 4,38 | | 2,96 | 2,64 | 2,65 | | |
| N511498 | 2,24 | 0,46 | 0,81 | 0,71 | 1,03 | 0,78 | 0,51 |
| N520547 | 7,74 | | 3,03 | 3,74 | 2,70 | | |
| N527039 | 2,36 | 0,31 | 0,79 | 0,41 | 1,03 | 0,70 | 0,60 |
| N530485 | 6,80 | 0,00 | 0,32 | 0,00 | 1,40 | 0,00 | 0,66 |
| N593116 | 2,38 | 0,99 | 1,41 | 1,95 | 1,42 | 1,49 | 1,46 |
| N734641 | 3,04 | | 8,30 | 7,31 | 7,23 | | |
| N748022 | 2,14 | 0 | 0,17 | 0,00 | 0,46 | 0,00 | 0,47 |
| N828353 | 8,21 | | 4,52 | 4,11 | 4,01 | | |
| N831245 | 7,74 | | 2,77 | 2,32 | 2,48 | | |
| N862300 | 3,59 | 1,79 | 4,75 | | 3,53 | | 2,33 |

Les corrélations de ces différents tests par rapport au test 2C9G6 / 5A8H2 sont rapportées dans le tableau 5 ci-dessous.

**Tableau 5**

| Données de corrélation entre les différents tests ELISA | | | | | | |
|---|---|---|---|---|---|---|
| Parameter | 5A8H2/3D6G1 | 2C9G6 / 2E5G9 | 3A7B4/3D6G1 | 3D6G1/5A8H2 | 8B10B8/5A8H2 | Hycult |
| Number of XY Pairs | 47 | 45 | 47 | 45 | 47 | 47 |
| Spearman r | 0,9864 | 0,9783 | 0,9244 | 0,9909 | 0,8091 | 0,5298 |
| 95% confidence interval | 0.9752 to 0.9926 | 0.9598 to 0.9883 | 0.8653 to 0.9581 | 0.9831 to 0.9951 | 0.6752 to 0.8914 | 0.2781 to 0.7135 |
| P value (two-tailed) | < 0.0001 | < 0.0001 | < 0.0001 | < 0.0001 | < 0.0001 | 0,0001 |
| Is the correlation significant? (alpha=0.05) | Yes | Yes | Yes | Yes | Yes | Yes |

Les différents tests développés en Vidas® ont une bonne corrélation entre eux et peuvent tous être utilisés pour doser la protéine LFABP.

### Exemple 3 : Détection des patients adénomes par le dosage ELISA de la LFABP sur l'automate Vidas®

### 1) Patients et prélèvements

La collecte des échantillons de sang a été réalisée grâce à un réseau de 8 centres cliniques répartis dans toute la France, dans le cadre de 2 protocoles loi Huriet pour les patients atteints de cancer colorectal. Pour les patients contrôles, coloscopies négatives, et porteurs d'adénomes colorectaux, une étude multicentrique impliquant une trentaine de gastroentérologues a été mise en place pour collecter le sang de patients Hémoccult™ positifs, avant la réalisation de la coloscopie et de toute préparation colique.

Tous les prélèvements ont été réalisés en conformité avec la législation en vigueur et les Bonnes Pratiques Cliniques, suivant un process de stockage et de suivi conformes aux bonnes pratiques de laboratoire, les patients et les contrôles coloscopies négatives étant prélevés exactement dans les mêmes conditions.

Pour l'obtention de sérum, le prélèvement sanguin se fait sur tube sec. Après coagulation, le tube est centrifugé 10 min à 1000 g, le sérum est prélevé, aliquoté et conservé à -80°C. Les échantillons sont parfaitement documentés pour l'histoire clinique des patients.

Au total, 147 patients ont été testés: 70 patients contrôle avec une coloscopie totalement normale (Coloneg), et 77 patients atteints d'adénomes colorectaux (dysplasie de haut grade ou de bas grade).

Parmi les 77 adénomes :
24 sont des adénomes à bas risque (dysplasie de bas grade et taille<1cm, et absence de composante villeuse)
37 sont des adénomes à haut risque sans dysplasie sévère (>= 1cm ou présence d'une composante villeuse)
16 sont des adénomes à haut risque haut grade (dysplasie sévère, quelle que soit la taille, avec ou sans composante villeuse).

### 2) Dosages Vidas® LFABP, ACE et CA19-9

La protéine LFABP a été dosée à l'aide du test LFABP Vidas® 2C9G6/5A8H2 décrit dans l'exemple 2. Ce type de test peut aussi être réalisé en microplaque, de façon automatisée ou manuelle.

Pour cet exemple, le test ELISA a été construit en utilisant les réactifs du kit Vidas® HBs Ag Ultra (bioMérieux, Cat. No. 30315).

Les réactifs ont été utilisés tels que décrits dans la notice correspondante (réf. 11728 D-FR-2005/05), avec les modifications suivantes :
- Les cônes ont été sensibilisés avec les l'Ac de capture 2C9G6 utilisé à une concentration de 10 µg/ml.
- Le contenu du deuxième puits de la cartouche HBS Ag Ultra a été remplacé par 300 µl d'anticorps de révélation 5A8H2, couplé à la biotine, dilués à 1 µg/ml dans le tampon du deuxième puits du kit Vidas® HBs Ag Ultra (tampon avec sérum de chèvre et azoture de sodium à 1 g/l).
- L'échantillon de sérum (50µl) a été dilué directement dans le deuxième puits de la cartouche HBS Ag Ultra.
- La réaction ELISA a été réalisée à l'aide de l'automate Vidas® et du protocole HBS dont l'étape d'incubation de l'échantillon avec les anticorps de capture et de révélation avait été porté à 100 cycles.
- Les résultats ont été obtenus sous forme de valeurs brutes après soustraction du bruit de fond (lecture du substrat avant réaction).

Les marqueurs tumoraux ACE et CA19-9 ont été dosés à l'aide des trousses de dosage de la Demanderesse, respectivement Vidas® CEAs, Vidas® CA19-9, en suivant le protocole opératoire propre à chaque trousse.

Une courbe étalon a été établie en dosant une gamme de concentration de la protéine biomarqueur recombinante. La courbe étalon a été tracée en reportant en abscisse la concentration de la protéine biomarqueur et en ordonnée le signal lu par Vidas® (RFV ou Relative Fluorescence Value). La concentration de la protéine biomarqueur présente dans le fluide corporel à doser (sang, sérum, plasma, selle) a été calculée en reportant la concentration correspondant au signal RFV lu par Vidas®.

**Tableau 6**

| Dosages sériques de laLFABP,de l'ACE et du CA-19-9 chez lespatientsadénomes et les contrôles coloscopies négatives (ColoNeg) | | | | | | | |
|---|---|---|---|---|---|---|---|
| Pathologie | N° Patient | Age | Sexe | Stade TNM | ACE ng/mL | CA19-9 U/mL | LFABP ng/ml |
| ColoNeg | HGED005 | 68 | Homme | NA | 2,08 | 4,38 | 3,70 |
| ColoNeg | PROMIS 21-01-005 | 68 | Homme | NA | 0,50 | 16,94 | 5,94 |
| ColoNeg | PROMIS 21-01-006 | 62 | Homme | NA | 0,00 | 2,10 | 2,65 |
| ColoNeg | PROMIS 21-01-009 | 51 | Femme | NA | 0,68 | 10,36 | 9,52 |
| ColoNeg | PROMIS 21-01-011 | 60 | Femme | NA | 0,00 | 4,31 | 0,45 |
| ColoNeg | PROMIS 21-01-012 | 55 | Femme | NA | 2,10 | 9,02 | 1,94 |
| ColoNeg | PROMIS 21-02-001 | 73 | Femme | NA | 1,15 | 5,02 | 11,06 |
| ColoNeg | PROMIS 21-02-003 | 55 | Femme | NA | 0,81 | 19,93 | 3,38 |
| ColoNeg | PROMIS 21-02-004 | 57 | Homme | NA | 0,00 | 2,48 | 7,23 |
| ColoNeg | PROMIS 21-02-006 | 51 | Homme | NA | 0,00 | 3,67 | 4,47 |
| ColoNeg | PROMIS 21-02-008 | 55 | Homme | NA | 1,10 | 7,61 | 10,22 |
| ColoNeg | PROMIS 21-02-011 | 55 | Femme | NA | 1,56 | 13,65 | 2,01 |
| ColoNeg | PROMIS 21-02-014 | 58 | Homme | NA | 0,81 | 2,02 | 6,68 |
| ColoNeg | PROMIS 21-02-016 | 62 | Homme | NA | 3,36 | 3,43 | 0,43 |
| ColoNeg | PROMIS 21-03-004 | 61 | Homme | NA | 3,46 | 2,33 | 8,62 |
| ColoNeg | PROMIS 21-03-013 | 52 | Femme | NA | 0,00 | 1,02 | 2,98 |
| ColoNeg | PROMIS 21-03-015 | 74 | Femme | NA | 0,90 | 2,48 | 7,26 |
| ColoNeg | PROMIS 21-04-002 | 67 | Homme | NA | 0,83 | 9,73 | 5,97 |
| ColoNeg | PROMIS 21-04-006 | 72 | Homme | NA | 0,68 | 2,33 | 2,25 |
| ColoNeg | PROMIS 21-04-007 | 68 | Femme | NA | 0,79 | 3,04 | 7,26 |
| ColoNeg | PROMIS 21-04-008 | 62 | Femme | NA | 1,06 | 1,02 | 5,97 |
| ColoNeg | PROMIS 21-04-009 | 58 | Homme | NA | 1,10 | 11,92 | 3,67 |
| ColoNeg | PROMIS 21-04-013 | 63 | Femme | NA | 3,09 | 11,92 | 5,03 |
| ColoNeg | PROMIS 21-07-004 | 66 | Homme | NA | 1,33 | 3,83 | 9,55 |
| ColoNeg | PROMIS 21-12-007 | 53 | Femme | NA | 0,00 | 2,86 | 6,03 |
| ColoNeg | PROMIS 21-12-011 | 52 | Homme | NA | 4,24 | 8,63 | 1,79 |
| ColoNeg | PROMIS 21-22-001 | 61 | Femme | NA | 2,87 | 17,25 | 2,08 |
| ColoNeg | PROMIS 21-22-006 | 65 | Femme | NA | 1,06 | 2,33 | 6,60 |
| ColoNeg | PROMIS 21-22-008 | 57 | Femme | NA | 0,00 | 2,79 | 5,20 |
| ColoNeg | PROMIS 37-03-004 | 53 | Femme | NA | 0,74 | 2,17 | 2,18 |
| ColoNeg | PROMIS 37-04-001 | 62 | Homme | NA | 5,16 | 4,54 | 7,09 |
| ColoNeg | PROMIS 37-04-005 | 69 | Femme | NA | 0,92 | 3,51 | 6,51 |
| ColoNeg | PROMIS 37-05-008 | 55 | Homme | NA | 0,00 | 2,86 | 6,60 |
| ColoNeg | PROMIS 37-06-001 | 65 | Femme | NA | 1,49 | 5,65 | 5,99 |
| ColoNeg | PROMIS 37-09-002 | 50 | Femme | NA | 0,77 | 26,00 | 1,74 |
| ColoNeg | PROMIS 37-11-001 | 56 | Homme | NA | 0,00 | 1,63 | 2,72 |
| ColoNeg | PROMIS 37-11-002 | 59 | Homme | NA | 0,00 | 1,86 | 2,40 |
| ColoNeg | PROMIS 37-11-011 | 71 | Homme | NA | 1,28 | 0,48 | 4,69 |
| ColoNeg | PROMIS 37-11-016 | 50 | Femme | NA | 0,00 | 5,02 | 1,87 |
| ColoNeg | PROMIS 37-12-002 | 63 | Homme | NA | 0,79 | 2,63 | 3,01 |
| ColoNeg | PROMIS 37-13-003 | 53 | Femme | NA | 0,92 | 7,38 | 4,28 |
| ColoNeg | PROMIS 37-13-006 | 53 | Homme | NA | 0,88 | 1,48 | 8,85 |
| ColoNeg | PROMIS 37-13-008 | 61 | Homme | NA | 0,00 | 0,94 | 4,22 |
| ColoNeg | PROMIS 37-14-009 | 63 | Homme | NA | 0,97 | 0,64 | 8,69 |
| ColoNeg | PROMIS 37-14-018 | 60 | Homme | NA | 0,57 | 2,86 | 6,11 |
| ColoNeg | PROMIS 37-14-021 | 61 | Femme | NA | 0,92 | 3,59 | 0,62 |
| ColoNeg | PROMIS 37-15-002 | 68 | Femme | NA | 0,68 | 3,75 | 3,15 |
| ColoNeg | PROMIS 37-15-006 | 67 | Femme | NA | 1,08 | 2,10 | 6,30 |
| ColoNeg | PROMIS 37-16-001 | 57 | Homme | NA | 0,74 | 3,75 | 3,99 |
| ColoNeg | PROMIS 37-17-001 | 52 | Homme | NA | 1,87 | 4,78 | 4,63 |
| ColoNeg | PROMIS 37-17-003 | 55 | Homme | NA | 5,81 | 1,71 | 2,65 |
| ColoNeg | PROMIS 37-17-004 | 58 | Femme | NA | 1,01 | 3,04 | 5,68 |
| ColoNeg | PROMIS 37-18-001 | 53 | Homme | NA | 0,55 | 3,43 | 4,31 |
| ColoNeg | PROMIS 37-18-003 | 50 | Femme | NA | 0,63 | 2,33 | 7,76 |
| ColoNeg | PROMIS 37-18-004 | 57 | Homme | NA | 0,00 | 2,10 | 3,83 |
| ColoNeg | PROMIS 37-18-005 | 55 | Femme | NA | 0,85 | 3,28 | 3,49 |
| ColoNeg | PROMIS 37-18-024 | | Homme | NA | 1,10 | 4,38 | 2,55 |
| ColoNeg | PROMIS 37-19-006 | 50 | Homme | NA | 0,00 | 1,63 | 9,08 |
| ColoNeg | PROMIS 37-19-007 | 51 | Femme | NA | 0,66 | 0,56 | 3,33 |
| ColoNeg | PROMIS 37-20-004 | 53 | Femme | NA | 1,17 | 1,02 | 2,84 |
| ColoNeg | PROMIS 71-02-003 | 60 | Femme | NA | 0,55 | 1,33 | 1,62 |
| ColoNeg | PROMIS 71-04-003 | 75 | Femme | NA | 0,57 | 5,57 | 6,93 |
| ColoNeg | PROMIS 71-09-007 | 52 | Homme | NA | 0,88 | 4,86 | 4,81 |
| ColoNeg | PROMIS 71-10-001 | 56 | Femme | NA | 1,15 | 15,14 | 2,15 |
| ColoNeg | PROMIS 71-10-007 | 71 | Femme | NA | 2,80 | 3,20 | 0,82 |
| ColoNeg | PROMIS 71-11-001 | 63 | Homme | NA | 1,45 | 3,00 | 3,44 |
| ColoNeg | PROMIS 71-11-003 | 59 | Femme | NA | 1,26 | 40,93 | 3,91 |
| ColoNeg | PROMIS 71-11-006 | 59 | Homme | NA | 0,66 | 2,33 | 4,22 |
| ColoNeg | PROMIS 71-11-008 | 54 | Homme | NA | 0,88 | 8,32 | 2,59 |
| ColoNeg | PROMIS 71-11-009 | 60 | Femme | NA | 0,50 | 7,30 | 5,82 |
| Adénome | GHAB007 | 67 | Homme | NA | 2,65 | 6,89 | 4,12 |
| Adénome | GHAB028 | 77 | Homme | NA | 1,79 | 3,53 | 13,02 |
| Adénome | GHAB046 | 54 | Homme | NA | 3,07 | 5,97 | 5,09 |
| Adénome | GHAB054 | 62 | Homme | NA | 1,22 | 3,00 | 2,09 |
| Adénome | GHBD079 | 76 | Homme | NA | 5,87 | 8,08 | 3,49 |
| Adénome | HGED009 | 68 | Femme | NA | 1,45 | 4,55 | 4,12 |
| Adénome | HGED013 | 59 | Homme | NA | 1,18 | 3,00 | 4,65 |
| Adénome | HGED019 | 52 | Homme | NA | 1,67 | 3,00 | 1,76 |
| Adénome | HGED038 | 58 | Homme | NA | 2,18 | 3,00 | 11,29 |
| Adénome | HGED046 | 57 | Homme | NA | 2,09 | 3,00 | 20,40 |
| Adénome | PROMIS 21-01-008 | | | NA | 0,55 | 3,00 | 2,14 |
| Adénome | PROMIS 21-02-012 | 50 | Homme | NA | 1,20 | 3,00 | 13,31 |
| Adénome | PROMIS 21-08-004 | 69 | Femme | NA | 1,26 | 3,00 | 2,65 |
| Adénome | PROMIS 21-22-016 | 68 | Femme | NA | 0,76 | 3,00 | 3,63 |
| Adénome | PROMIS 21-22-019 | 68 | Femme | NA | 0,50 | 3,28 | 3,88 |
| Adénome | PROMIS 37-03-008 | 53 | Homme | NA | 1,91 | 3,00 | 12,35 |
| Adénome | PROMIS 37-03-011 | 62 | Homme | NA | 0,50 | 3,00 | 4,92 |
| Adénome | PROMIS 37-05-012 | 71 | Femme | NA | 0,78 | 3,00 | 3,57 |
| Adénome | PROMIS 37-08-001 | 64 | Femme | NA | 1,24 | 3,00 | 9,82 |
| Adénome | PROMIS 37-09-001 | 56 | Femme | NA | 7,08 | 3,00 | 3,63 |
| Adénome | PROMIS 37-11-003 | 70 | Homme | NA | 2,14 | 3,00 | 6,83 |
| Adénome | PROMIS 37-11-008 | 51 | Homme | NA | 1,06 | 3,53 | 3,14 |
| Adénome | PROMIS 37-11-015 | 69 | Homme | NA | 2,67 | 3,00 | 10,56 |
| Adénome | PROMIS 37-11-018 | 57 | Homme | NA | 1,61 | 29,05 | 7,95 |
| Adénome | PROMIS 37-13-002 | 59 | Homme | NA | 1,71 | 3,00 | 4,65 |
| Adénome | PROMIS 37-14-002 | 53 | Homme | NA | 1,57 | 3,00 | 3,73 |
| Adénome | PROMIS 37-14-003 | 63 | Femme | NA | 1,45 | 3,00 | 8,05 |
| Adénome | PROMIS 37-14-008 | 52 | Femme | NA | 0,55 | 3,00 | 0,89 |
| Adénome | PROMIS 37-15-004 | 58 | Homme | NA | 3,18 | 9,30 | 1,59 |
| Adénome | PROMIS 37-15-005 | 72 | Homme | NA | 3,07 | 3,00 | 0,68 |
| Adénome | PROMIS 37-20-003 | 64 | Homme | NA | 2,91 | 3,00 | 13,31 |
| Adénome | PROMIS 37-23-001 | 52 | Homme | NA | 3,84 | 9,92 | 4,72 |
| Adénome | PROMIS 37-23-004 | 71 | Femme | NA | 1,08 | 10,20 | 4,31 |
| Adénome | PROMIS 71-08-008 | 51 | Homme | NA | 1,24 | 3,00 | 7,08 |
| Adénome | PROMIS 71-10-002 | 62 | Homme | NA | 2,37 | 18,29 | 1,09 |
| Adénome | PROMIS 71-10-004 | 63 | Homme | NA | 2,01 | 3,77 | 3,04 |
| Adénome | PROMIS 71-11-002 | 75 | Homme | NA | 2,47 | 6,02 | 26,32 |
| Adénome | CLSP350 | 76 | Homme | NA | 7,03 | 3,00 | 3,05 |
| Adénome | CLSP398 | 43 | Femme | NA | 1,32 | 3,00 | 4,82 |
| Adénome | CLSP427 | 83 | Homme | NA | 3,11 | 7,99 | 11,24 |
| Adénome | CLSP457 | 66 | Homme | NA | 10,92 | 3,00 | 11,36 |
| Adénome | CLSP681 | 61 | Femme | NA | 0,76 | 8,27 | 26,13 |
| Adénome | CLSP769 | 50 | Femme | NA | 0,50 | 3,00 | 44,76 |
| Adénome | GHBD014 | 71 | Homme | NA | 1,95 | 4,13 | 24,03 |
| Adénome | GHBD053 | 66 | Femme | NA | 0,85 | 3,00 | 4,56 |
| Adénome | PROMIS 21-02-007 | | | NA | 0,61 | 3,00 | 8,56 |
| Adénome | PROMIS 21-02-009 | | | NA | 3,22 | 7,93 | 7,09 |
| Adénome | PROMIS 21-02-017 | 64 | Homme | NA | 1,49 | 3,00 | 1,83 |
| Adénome | PROMIS 21-03-001 | 58 | Homme | NA | 2,09 | 3,00 | 7,32 |
| Adénome | PROMIS 21-03-017 | 69 | Homme | NA | 1,57 | 5,44 | 4,52 |
| Adénome | PROMIS 21-08-001 | 52 | Homme | NA | 3,26 | 11,66 | 3,71 |
| Adénome | PROMIS 37-03-003 | 57 | Homme | NA | 0,97 | 3,00 | 5,98 |
| Adénome | PROMIS 37-03-006 | 60 | Femme | NA | 0,50 | 14,27 | 0,50 |
| Adénome | PROMIS 37-06-003 | 60 | Femme | NA | 0,64 | 3,00 | 1,82 |
| Adénome | PROMIS 37-09-004 | 74 | Homme | NA | 2,18 | 5,61 | 10,20 |
| Adénome | PROMIS 37-11-005 | 54 | Femme | NA | 0,53 | 3,00 | 3,20 |
| Adénome | PROMIS 37-13-007 | 65 | Femme | NA | 0,93 | 3,00 | 3,53 |
| Adénome | PROMIS 37-14-015 | 74 | Femme | NA | 1,45 | 8,39 | 1,88 |
| Adénome | PROMIS 37-19-001 | 71 | Homme | NA | 1,77 | 5,44 | 4,53 |
| Adénome | PROMIS 37-23-003 | 67 | Femme | NA | 0,82 | 5,67 | 5,18 |
| Adénome | PROMIS 71-10-012 | 50 | Homme | NA | 0,66 | 3,00 | 12,36 |
| Adénome | CLSP022 | 70 | Homme | NA | 200,00 | 3,00 | 0,71 |
| Adénome | CLSP036 | 70 | Homme | NA | 0,91 | 3,00 | 7,73 |
| Adénome | CLSP270 | 75 | Homme | NA | 1,20 | 3,00 | 10,36 |
| Adénome | CLSP287 | 56 | Femme | NA | 1,20 | 3,16 | 2,83 |
| Adénome | CLSP337 | 63 | Femme | NA | 0,61 | 1,71 | 14,47 |
| Adénome | CLSP358 | 73 | Homme | NA | 2,09 | 22,23 | 5,48 |
| Adénome | CLSP359 | 60 | Homme | NA | 1,65 | 17,96 | 6,58 |
| Adénome | CLSP362 | 81 | Homme | NA | 1,28 | 3,00 | 11,19 |
| Adénome | CLSP378 | 75 | Homme | NA | 0,63 | 2,10 | 3,93 |
| Adénome | CLSP719 | 62 | Homme | NA | 1,55 | 3,00 | 19,42 |
| Adénome | CNSE018 | 75 | Femme | NA | 0,50 | 3,00 | 7,42 |
| Adénome | GESE003 | 59 | Femme | NA | 0,50 | 3,00 | 2,46 |
| Adénome | GHAB004 | 84 | Homme | NA | 4,17 | 4,79 | 5,80 |
| Adénome | GHBD002 | 87 | Homme | NA | 0,61 | 3,00 | 4,28 |
| Adénome | PROMIS 21-22-004 | 71 | Femme | NA | 2,73 | 21,25 | 3,62 |
| Adénome | PROMIS 71-10-006 | 57 | Homme | NA | 4,74 | 18,84 | 9,59 |
| | Seuil (concentration max des ColoNeg) | | | | 5,81 | 40,93 | 11,06 |
| | Adénomes supérieurs au seuil | | | | 4 | 0 | 16 |

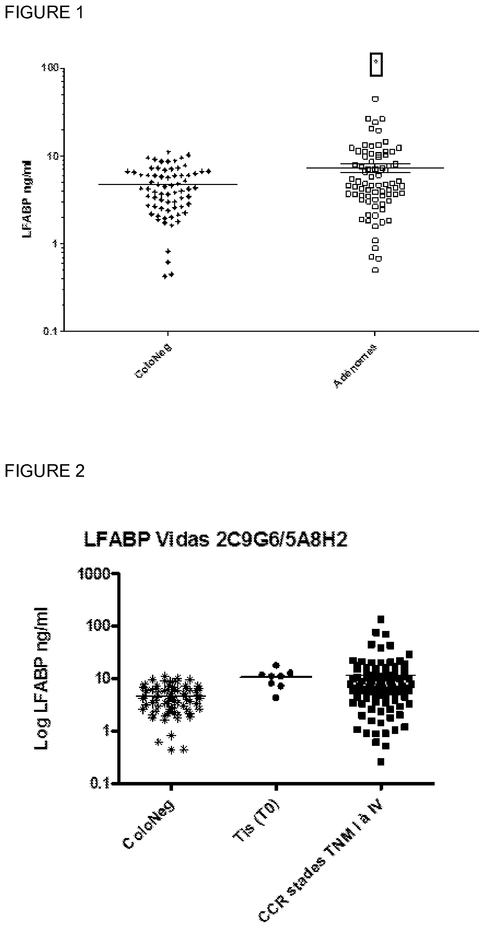

Les résultats du dosage de la LFABP sont représentés sur la Figure 1.

Avec le seuil de LFABP de 11,06 ng/ml (taux le plus élevé pour les contrôles ColoNeg, soit une spécificité de 100%), on détecte 16 patients adénome sur 77, soit 20.5% de sensibilité pour les adénomes, haut risque et bas risque confondus. Le haut risque étant défini par la taille >=1cm, la présence d'une dysplasie sévère ou la présence d'une composante villeuse.

Pour connaître la valeur prédictive qu'aurait un test de détection des adénomes, il faut avoir la prévalence de la maladie.

Suivant les études (autopsie, coloscopies systématiques, évaluation post sang dans les selles), la prévalence des adénomes varie un peu. Dans une étude récente publiée dans le JAMA [Ferlitsch et al, Sex-Specific Prevalence of Adenomas, Advanced Adenomas, and Colorectal Cancer in Individuals Undergoing Screening Colonoscopy JAMA, September 28, 2011-Vol 306, No. 12], la prévalence de tous les adénomes est décrite à 19.7%, et celle des adénomes avancés à 6.3%. mais dans cette même étude la prévalence des cancers est de 1.1%, ce qui est le double de ce qui est décrit en Europe (peut-être du fait d'un dépistage par coloscopie : pas que des sujets asymptomatiques). La définition des adénomes avancés est peut être différente de celle que nous avons utilisée.

Dans une étude autopsique [Williams AR, Balasooriya BA, Day DW. Polyps and cancer of the large bowel: a necropsy study in Liverpool. Gut. 1982 Oct;23(10):835-42], la prévalence avait été décrite à 33% pour les adénomes et 13 % pour les adénomes avancés.

Sur la base des résultats ci-dessus, en fixant le seuil à 11.06 ng/ml pour garder le même seuil que pour les carcinomes in situ T0, on détecte 20.5% des adénomes. On peut extrapoler une valeur prédictive positive du test LFABP pour la détection des adénomes : de 50% à 62% suivant les chiffres de prévalence utilisés (pour une spécificité fixée à 95%).

Donc, la valeur prédictive positive (VPP) du dosage de la LFABP dans le sérum des patients, pour la détection des adénomes, est bonne. En particulier la VPP est aussi bonne que ce qui est décrit pour les tests de sang dans les selles (les chiffres de VPP dans la littérature sont de 34 à 43% pour les adénomes avancés en fonction du test de recherche de sang dans les selles utilisé, sur les études de dépistage réalisées avec les tests immunologiques de recherche de sang dans les selles, et autour de 13% à 20% pour les tests au gaïac).

Résultats des dosages de ACE (sur Vidas® CEAs) pour la détection des patients adénomes :
Comparativement, seulement 4 patients adénomes ont un taux d'ACE au-dessus du seuil des contrôles coloscopie négatives (6.05 ng/ml dans notre cohorte, décrit pour être entre 5 et 10 ng/ml). Seuls 2 patients ont un taux d'ACE>10ng/ml sur les 77 patients adénomes testés.

Résultats des dosages de CA19-9 (sur Vidas® CA19-0) pour la détection des patients adénomes :
Il n'y a pas de patient adénome qui ait un taux de CA-19-9 au-dessus du seuil des coloscopies négatives. Pour les patients adénomes, le taux le plus élevé est de 29,05 U/ml.

### Exemple 4 : Détection des tumeurs in situ (T0) par le dosage ELISA de la LFABP sur l'automate Vidas®

### 1) Patients et prélèvements

La collecte des échantillons de sang a été réalisée grâce à un réseau de 8 centres cliniques répartis dans toute la France, dans le cadre de 2 protocoles loi Huriet pour les patients atteints de cancer colorectal et de tumeurs in situ. Pour les patients contrôles, coloscopies négatives, et porteurs d'adénomes colorectaux, une étude multicentrique impliquant une trentaine de gastroentérologues a été mise en place pour collecter le sang de patients Hémoccult™ positifs, avant la réalisation de la coloscopie et de toute préparation colique. Par ailleurs, des échantillons de sérum ont été obtenus auprès de donneurs de sang des établissements français du sang (EFS), suivant les procédures en pratique dans ces centres.

Tous les prélèvements ont été réalisés en conformité avec la législation en vigueur et les Bonnes Pratiques Cliniques, suivant un process de stockage et de suivi conformes aux bonnes pratiques de laboratoire, les patients et les contrôles coloscopies négatives étant prélevés exactement dans les mêmes conditions.

Pour l'obtention de sérum, le prélèvement sanguin se fait sur tube sec. Après coagulation, le tube est centrifugé 10 min à 1000 g, le sérum est prélevé, aliquoté et conservé à -80°C. Les échantillons sont parfaitement documentés pour l'histoire clinique des patients.

Au total, 182 patients ont été testés : 8 patients présentant une tumeur in situ T0, 104 patients présentant un cancer colorectal invasif de stade TNM I à IV, 70 patients contrôles avec une coloscopie totalement normale.
- 8 patients atteints de Tis (Tumeur in situ, ou carcinome intra-muqueux, non invasif (T0, N0, MO au moment du prélèvement sanguin)
- 14 patients CCR invasif de stade TNM I
- 49 patients CCR invasif de stade TNM II
- 27 patients CCR invasif de stade TNM III
- 13 patients CCR invasif de stade TNM IV
- 1 patient CCR non classé

### 2) Dosages Vidas®

La protéine LFABP a été dosée à l'aide du test LFABP Vidas® 2C9G6/5A8H2 décrit dans l'exemple 2. Ce type de test peut aussi être réalisé en microplaque, de façon automatisée ou manuelle.

Pour cet exemple, le test ELISA a été construit en utilisant les réactifs du kit Vidas® HBs Ag Ultra (bioMérieux, Cat. No. 30315). Les réactifs ont été utilisés tels que décrits dans la notice correspondante (réf. 11728 D-FR-2005/05), modifiée comme suit :
- Les cônes ont été sensibilisés avec les l'Ac de capture 2C9G6 utilisé à une concentration de 10 µg/ml.
- Le contenu du deuxième puits de la cartouche HBS Ag Ultra a été remplacé par 300 µl d'anticorps de révélation 5A8H2, couplé à la biotine, dilués à 1 µg/ml dans le tampon du deuxième puits du kit Vidas® HBs Ag Ultra (tampon avec sérum de chèvre et azoture de sodium à 1 g/l).
- L'échantillon de sérum (50µl) a été dilué directement dans le deuxième puits de la cartouche HBS Ag Ultra.
- La réaction ELISA a été réalisée à l'aide de l'automate Vidas® et du protocole HBS dont l'étape d'incubation de l'échantillon avec les anticorps de capture et de révélation avait été porté à 100 cycles.
- Les résultats ont été obtenus sous forme de valeurs brutes après soustraction du bruit de fond (lecture du substrat avant réaction).

Les marqueurs tumoraux ACE et CA19-9 ont été dosés à l'aide des trousses de dosage de la Demanderesse, respectivement Vidas® CEAs, Vidas® CA19-9, en suivant le protocole opératoire propre à chaque trousse.

Une courbe étalon a été établie en dosant une gamme de concentration de la protéine biomarqueur recombinante. La courbe étalon a été tracée en reportant en abscisse la concentration de la protéine biomarqueur et en ordonnée le signal lu par Vidas® (RFV ou Relative Fluorescence Value). La concentration de la protéine biomarqueur présente dans le fluide corporel à doser (sang, sérum, plasma, selle) a été calculée en reportant la concentration correspondant au signal RFV lu par Vidas®.

Les marqueurs tumoraux ACE et CA19-9 ont été dosés à l'aide des trousses de dosage de la Demanderesse, respectivement Vidas® CEAs, Vidas® CA19-9, en suivant le protocole opératoire propre à chaque trousse.

**Tableau 7**

| Dosages LFABP, ACE et CA19-9 des 182 patients testés | | | | | | | | |
|---|---|---|---|---|---|---|---|---|
| Pathologie | N° Patient | Age | Sexe | Stade TNM | Note | ACE ng/mL | CA19-9 U/mL | LFABP ng/ml |
| ColoNeg | HGED005 | 68 | Homme | NA | | 2,08 | 4,38 | 3,70 |
| ColoNeg | PROMIS 21-01-005 | 68 | Homme | NA | | 0,50 | 16,94 | 5,94 |
| ColoNeg | PROMIS 21-01-006 | 62 | Homme | NA | | 0,00 | 2,10 | 2,65 |
| ColoNeg | PROMIS 21-01-009 | 51 | Femme | NA | | 0,68 | 10,36 | 9,52 |
| ColoNeg | PROMIS 21-01-011 | 60 | Femme | NA | | 0,00 | 4,31 | 0,45 |
| ColoNeg | PROMIS 21-01-012 | 55 | Femme | NA | | 2,10 | 9,02 | 1,94 |
| ColoNeg | PROMIS 21-02-001 | 73 | Femme | NA | | 1,15 | 5,02 | 11,06 |
| ColoNeg | PROMIS 21-02-003 | 55 | Femme | NA | | 0,81 | 19,93 | 3,38 |
| ColoNeg | PROMIS 21-02-004 | 57 | Homme | NA | | 0,00 | 2,48 | 7,23 |
| ColoNeg | PROMIS 21-02-006 | 51 | Homme | NA | | 0,00 | 3,67 | 4,47 |
| ColoNeg | PROMIS 21-02-008 | 55 | Homme | NA | | 1,10 | 7,61 | 10,22 |
| ColoNeg | PROMIS 21-02-011 | 55 | Femme | NA | | 1,56 | 13,65 | 2,01 |
| ColoNeg | PROMIS 21-02-014 | 58 | Homme | NA | | 0,81 | 2,02 | 6,68 |
| ColoNeg | PROMIS 21-02-016 | 62 | Homme | NA | | 3,36 | 3,43 | 0,43 |
| ColoNeg | PROMIS 21-03-004 | 61 | Homme | NA | | 3,46 | 2,33 | 8,62 |
| ColoNeg | PROMIS 21-03-013 | 52 | Femme | NA | | 0,00 | 1,02 | 2,98 |
| ColoNeg | PROMIS 21-03-015 | 74 | Femme | NA | | 0,90 | 2,48 | 7,26 |
| ColoNeg | PROMIS 21-04-002 | 67 | Homme | NA | | 0,83 | 9,73 | 5,97 |
| ColoNeg | PROMIS 21-04-006 | 72 | Homme | NA | | 0,68 | 2,33 | 2,25 |
| ColoNeg | PROMIS 21-04-007 | 68 | Femme | NA | | 0,79 | 3,04 | 7,26 |
| ColoNeg | PROMIS 21-04-008 | 62 | Femme | NA | | 1,06 | 1,02 | 5,97 |
| ColoNeg | PROMIS 21-04-009 | 58 | Homme | NA | | 1,10 | 11,92 | 3,67 |
| ColoNeg | PROMIS 21-04-013 | 63 | Femme | NA | | 3,09 | 11,92 | 5,03 |
| ColoNeg | PROMIS 21-07-004 | 66 | Homme | NA | | 1,33 | 3,83 | 9,55 |
| ColoNeg | PROMIS 21-12-007 | 53 | Femme | NA | | 0,00 | 2,86 | 6,03 |
| ColoNeg | PROMIS 21-12-011 | 52 | Homme | NA | | 4,24 | 8,63 | 1,79 |
| ColoNeg | PROMIS 21-22-001 | 61 | Femme | NA | | 2,87 | 17,25 | 2,08 |
| ColoNeg | PROMIS 21-22-006 | 65 | Femme | NA | | 1,06 | 2,33 | 6,60 |
| ColoNeg | PROMIS 21-22-008 | 57 | Femme | NA | | 0,00 | 2,79 | 5,20 |
| ColoNeg | PROMIS 37-03-004 | 53 | Femme | NA | | 0,74 | 2,17 | 2,18 |
| ColoNeg | PROMIS 37-04-001 | 62 | Homme | NA | | 5,16 | 4,54 | 7,09 |
| ColoNeg | PROMIS 37-04-005 | 69 | Femme | NA | | 0,92 | 3,51 | 6,51 |
| ColoNeg | PROMIS 37-05-008 | 55 | Homme | NA | | 0,00 | 2,86 | 6,60 |
| ColoNeg | PROMIS 37-06-001 | 65 | Femme | NA | | 1,49 | 5,65 | 5,99 |
| ColoNeg | PROMIS 37-09-002 | 50 | Femme | NA | | 0,77 | 26,00 | 1,74 |
| ColoNeg | PROMIS 37-11-001 | 56 | Homme | NA | | 0,00 | 1,63 | 2,72 |
| ColoNeg | PROMIS 37-11-002 | 59 | Homme | NA | | 0,00 | 1,86 | 2,40 |
| ColoNeg | PROMIS 37-11-011 | 71 | Homme | NA | | 1,28 | 0,48 | 4,69 |
| ColoNeg | PROMIS 37-11-016 | 50 | Femme | NA | | 0,00 | 5,02 | 1,87 |
| ColoNeg | PROMIS 37-12-002 | 63 | Homme | NA | | 0,79 | 2,63 | 3,01 |
| ColoNeg | PROMIS 37-13-003 | 53 | Femme | NA | | 0,92 | 7,38 | 4,28 |
| ColoNeg | PROMIS 37-13-006 | 53 | Homme | NA | | 0,88 | 1,48 | 8,85 |
| ColoNeg | PROMIS 37-13-008 | 61 | Homme | NA | | 0,00 | 0,94 | 4,22 |
| ColoNeg | PROMIS 37-14-009 | 63 | Homme | NA | | 0,97 | 0,64 | 8,69 |
| ColoNeg | PROMIS 37-14-018 | 60 | Homme | NA | | 0,57 | 2,86 | 6,11 |
| ColoNeg | PROMIS 37-14-021 | 61 | Femme | NA | | 0,92 | 3,59 | 0,62 |
| ColoNeg | PROMIS 37-15-002 | 68 | Femme | NA | | 0,68 | 3,75 | 3,15 |
| ColoNeg | PROMIS 37-15-006 | 67 | Femme | NA | | 1,08 | 2,10 | 6,30 |
| ColoNeg | PROMIS 37-16-001 | 57 | Homme | NA | | 0,74 | 3,75 | 3,99 |
| ColoNeg | PROMIS 37-17-001 | 52 | Homme | NA | | 1,87 | 4,78 | 4,63 |
| ColoNeg | PROMIS 37-17-003 | 55 | Homme | NA | | 5,81 | 1,71 | 2,65 |
| ColoNeg | PROMIS 37-17-004 | 58 | Femme | NA | | 1,01 | 3,04 | 5,68 |
| ColoNeg | PROMIS 37-18-001 | 53 | Homme | NA | | 0,55 | 3,43 | 4,31 |
| ColoNeg | PROMIS 37-18-003 | 50 | Femme | NA | | 0,63 | 2,33 | 7,76 |
| ColoNeg | PROMIS 37-18-004 | 57 | Homme | NA | | 0,00 | 2,10 | 3,83 |
| ColoNeg | PROMIS 37-18-005 | 55 | Femme | NA | | 0,85 | 3,28 | 3,49 |
| ColoNeg | PROMIS 37-18-024 | | Homme | NA | | 1,10 | 4,38 | 2,55 |
| ColoNeg | PROMIS 37-19-006 | 50 | Homme | NA | | 0,00 | 1,63 | 9,08 |
| ColoNeg | PROMIS 37-19-007 | 51 | Femme | NA | | 0,66 | 0,56 | 3,33 |
| ColoNeg | PROMIS 37-20-004 | 53 | Femme | NA | | 1,17 | 1,02 | 2,84 |
| ColoNeg | PROMIS 71-02-003 | 60 | Femme | NA | | 0,55 | 1,33 | 1,62 |
| ColoNeg | PROMIS 71-04-003 | 75 | Femme | NA | | 0,57 | 5,57 | 6,93 |
| ColoNeg | PROMIS 71-09-007 | 52 | Homme | NA | | 0,88 | 4,86 | 4,81 |
| ColoNeg | PROMIS 71-10-001 | 56 | Femme | NA | | 1,15 | 15,14 | 2,15 |
| ColoNeg | PROMIS 71-10-007 | 71 | Femme | NA | | 2,80 | 3,20 | 0,82 |
| ColoNeg | PROMIS 71-11-001 | 63 | Homme | NA | | 1,45 | 3,00 | 3,44 |
| ColoNeg | PROMIS 71-11-003 | 59 | Femme | NA | | 1,26 | 40,93 | 3,91 |
| ColoNeg | PROMIS 71-11-006 | 59 | Homme | NA | | 0,66 | 2,33 | 4,22 |
| ColoNeg | PROMIS 71-11-008 | 54 | Homme | NA | | 0,88 | 8,32 | 2,59 |
| ColoNeg | PROMIS 71-11-009 | 60 | Femme | NA | | 0,50 | 7,30 | 5,82 |
| Tis | CLSP234 | 79 | Homme | T0 | | 1,45 | 2,33 | 11,29 |
| Tis | CLSP300 | 80 | Femme | T0 | | 1,61 | 2,02 | 8,15 |
| Tis | CLSP315 | 58 | Homme | T0 | | 0,70 | 2,10 | 11,12 |
| Tis | CLSP367 | 78 | Homme | T0 | | 0,90 | 4,31 | 7,21 |
| Tis | CLSP724 | 48 | Homme | T0 | | 0,92 | 28,45 | 11,90 |
| Tis | CNSE003 | 83 | Femme | T0 | | 0,00 | 5,65 | 17,82 |
| Tis | CNSE004 | 73 | Femme | T0 | | 0,00 | 1,40 | 4,30 |
| Tis | GHBD015 | 82 | Homme | T0 | | 0,83 | 5,65 | 12,83 |
| CCR | CBSE001 | 69 | Femme | I | | 4,06 | 3,00 | 14,92 |
| CCR | CBSE016 | 74 | Homme | I | | 6,79 | 1,25 | 17,36 |
| CCR | CLSP047 | 76 | Femme | I | | 1,06 | 0,79 | 0,26 |
| CCR | CLSP162 | 63 | Femme | I | | 0,00 | 0,71 | 6,22 |
| CCR | CLSP196 | 55 | Homme | I | | 3,71 | 8,95 | 10,30 |
| CCR | CLSP224 | 84 | Femme | I | | 4,62 | 6,12 | 42,38 |
| CCR | CLSP235 | 71 | Femme | I | | 0,00 | 3,43 | 2,63 |
| CCR | CLSP263 | 70 | Homme | I | | 1,10 | 30,04 | 8,05 |
| CCR | CLSP340 | 78 | Homme | I | | 1,28 | 3,00 | 3,85 |
| CCR | CLSP344 | 77 | Femme | I | | 1,49 | 2,17 | 3,78 |
| CCR | CLSP364 | 81 | Femme | I | | 1,24 | 4,62 | 1,42 |
| CCR | CLSP376 | 51 | Homme | I | | 1,17 | 12,63 | 2,58 |
| CCR | GHBD003 | 54 | Homme | I | | 0,99 | 2,56 | 0,88 |
| CCR | GHBD094 | 82 | Homme | I | | 1,12 | 9,18 | 8,08 |
| CCR | CLSP075 | 65 | Homme | II | | 0,74 | 2,71 | 5,19 |
| CCR | CLSP076 | 82 | Femme | II | | 0,97 | 0,71 | 10,27 |
| CCR | CLSP080 | 74 | Femme | II | | 0,59 | 4,23 | 0,89 |
| CCR | CLSP096 | 79 | Femme | II | | 12,70 | 7,22 | 8,86 |
| CCR | CLSP110 | 67 | Femme | II | | 0,00 | 2,25 | 10,41 |
| CCR | CLSP117 | 78 | Femme | II | | 2,08 | 5,25 | 20,19 |
| CCR | CLSP130 | 81 | Homme | II | | 1,15 | 3,59 | 11,15 |
| CCR | CLSP133 | 78 | Femme | II | | 0,70 | 3,59 | 3,52 |
| CCR | CLSP143 | 76 | Femme | II | | 5,45 | 2,33 | 5,87 |
| CCR | CLSP147 | 83 | Homme | II | | 1,56 | 1,56 | 3,55 |
| CCR | CLSP154 | 76 | Femme | II | | 11,04 | 8,40 | 6,33 |
| CCR | CLSP155 | 81 | Femme | II | | 11,53 | 37,95 | 3,30 |
| CCR | CLSP157 | 45 | Femme | II | | 0,70 | 1,48 | 8,47 |
| CCR | CLSP165 | 52 | Homme | II | | 0,00 | 2,33 | 5,64 |
| CCR | CLSP170 | 80 | Femme | II | | 5,11 | 8,00 | 13,10 |
| CCR | CLSP173 | 49 | Femme | II | | 0,66 | 8,79 | 0,61 |
| CCR | CLSP177 | 81 | Homme | II | | 1,47 | 29,49 | 8,63 |
| CCR | CLSP178 | 55 | Homme | II | | 20,68 | 8,95 | 101,48 |
| CCR | CLSP179 | 69 | Femme | II | | 5,81 | 8,24 | 9,89 |
| CCR | CLSP186 | 69 | Inconnu | II | | 1,45 | 2,94 | 13,47 |
| CCR | CLSP194 | 70 | Homme | II | | 1,01 | 1,79 | 19,11 |
| CCR | CLSP201 | 75 | Homme | II | | 2,51 | 0,71 | 16,22 |
| CCR | CLSP207 | 72 | Femme | II | | 1,35 | 26,00 | 15,17 |
| CCR | CLSP209 | 38 | Femme | II | | 0,97 | 2,63 | 17,92 |
| CCR | CLSP220 | 81 | Femme | II | | 7,52 | 12,79 | 10,63 |
| CCR | CLSP222 | 81 | Homme | II | | 5,50 | 3,83 | 21,07 |
| CCR | CLSP236 | 69 | Homme | II | | 0,00 | 1,63 | 13,64 |
| CCR | CLSP253 | 61 | Femme | II | | 1,54 | 2,48 | 6,64 |
| CCR | CLSP256 | 86 | Femme | II | | 3,54 | 10,75 | 8,27 |
| CCR | CLSP280 | 62 | Homme | II | | 2,20 | 8,63 | 5,70 |
| CCR | CLSP296 | 56 | Homme | II | | 1,89 | 73,08 | 9,93 |
| CCR | CLSP310 | 81 | Homme | II | | 1,89 | 6,98 | 2,94 |
| CCR | CLSP327 | 84 | Homme | II | | 0,94 | 1,17 | 7,25 |
| CCR | CLSP333 | 87 | Homme | II | | 1,58 | 19,45 | 10,01 |
| CCR | CLSP341 | 45 | Homme | II | | 0,53 | 3,67 | 3,46 |
| CCR | CLSP346 | 66 | Homme | II | | 7,20 | 39,88 | 5,67 |
| CCR | CLSP347 | 49 | Femme | II | | 7,41 | 1,25 | 6,43 |
| CCR | CLSP380 | 60 | Homme | II | | 0,70 | 3,04 | 7,83 |
| CCR | CNSE002 | 62 | Femme | II | | 3,84 | 4,46 | 1,58 |
| CCR | GHBD021 | 71 | Femme | II | | 0,53 | 1,94 | 1,04 |
| CCR | GHBD043 | 71 | Homme | II | | 2,27 | 9,18 | 5,78 |
| CCR | GHBD064 | 83 | Femme | II | | 21,08 | 5,65 | 23,26 |
| CCR | GHBD066 | 82 | Femme | II | | 0,57 | 1,94 | 6,98 |
| CCR | GHBD075 | 58 | Homme | II | | 2,53 | 1,86 | 6,34 |
| CCR | GHBD087 | 62 | Femme | II | | 2,17 | 5,10 | 8,83 |
| CCR | GHBD090 | 59 | Homme | II | | 0,79 | 3,59 | 4,82 |
| CCR | GHBD096 | 62 | Femme | II | | 3,04 | 1,71 | 6,80 |
| CCR | GHBD100 | 75 | Homme | II | | 2,08 | 4,94 | 10,82 |
| CCR | GHBD103 | 68 | Homme | II | | 1,06 | 3,20 | 10,41 |
| CCR | CBSE023 | 76 | Homme | III | | 2,27 | 9,34 | 11,10 |
| CCR | CLSP044 | 81 | Homme | III | | 74,10 | 4,31 | 4,69 |
| CCR | CLSP074 | 79 | Femme | III | | 0,81 | 7,06 | 4,26 |
| CCR | CLSP078 | 72 | Femme | III | | 0,57 | 2,33 | 21,69 |
| CCR | CLSP081 | 78 | Femme | III | | 1,56 | 2,94 | 4,80 |
| CCR | CLSP098 | 61 | Homme | III | | 0,77 | 2,56 | 4,55 |
| CCR | CLSP174 | 77 | Homme | III | | 31,33 | 264,90 | 20,46 |
| CCR | CLSP227 | 52 | Homme | III | | 14,52 | 7,30 | 44,85 |
| CCR | CLSP233 | 59 | Homme | III | | 1,03 | 4,23 | 2,38 |
| CCR | CLSP255 | 42 | Femme | III | | 0,85 | 65,91 | 30,53 |
| CCR | CLSP289 | 52 | Homme | III | | 0,90 | 2,17 | 8,30 |
| CCR | CLSP320 | 70 | Homme | III | | 1,70 | 3,04 | 3,62 |
| CCR | CLSP324 | 53 | Homme | III | | 4,57 | 15,37 | 2,02 |
| CCR | CLSP383 | 78 | Homme | III | | 1,84 | 1,79 | 1,20 |
| CCR | CLSP384 | 37 | Homme | III | | 68,52 | 26,63 | 15,70 |
| CCR | CNSE001 | 78 | Homme | III | | 6,45 | 40,28 | 5,87 |
| CCR | CNSE016 | 65 | Homme | III | | 20,59 | 91,16 | 52,41 |
| CCR | CNSE017 | 77 | Femme | III | | 0,61 | 2,17 | 16,79 |
| CCR | CSEM015 | 65 | Femme | III | | 1,38 | 1,02 | 11,51 |
| CCR | CSEM029 | 63 | Femme | III | | 2,13 | 3,67 | 5,65 |
| CCR | GHBD004 | 58 | Homme | III | | 16,26 | 5,73 | 1,97 |
| CCR | GHBD017 | 70 | Homme | III | | 0,68 | 3,99 | 0,91 |
| CCR | GHBD034 | 48 | Femme | III | | 0,57 | 2,94 | 0,52 |
| CCR | GHBD042 | 77 | Femme | III | | 0,50 | 13,88 | 5,95 |
| CCR | GHBD045 | 73 | Homme | III | | 0,55 | 1,56 | 1,07 |
| CCR | GHBD089 | 54 | Femme | III | | 24,47 | 10,12 | 6,99 |
| CCR | GHBD105 | 79 | Homme | III | | 1,35 | 37,39 | 5,84 |
| CCR | CBSE012 | 37 | Femme | IV | | 4,06 | 44,73 | 4,48 |
| CCR | CBSE026 | 72 | Homme | IV | | 333,40 | 23878,00 | 28,35 |
| CCR | CLSP156 | 59 | Homme | IV | | 14,00 | 43,27 | 8,75 |
| CCR | CLSP159 | 69 | Femme | IV | | 44,38 | 164,91 | 9,74 |
| CCR | CLSP167 | 81 | Homme | IV | | 1,89 | 35,39 | 1,80 |
| CCR | CLSP260 | 57 | Femme | IV | | 29,97 | 17,73 | 70,03 |
| CCR | CLSP334 | 54 | Femme | IV | | 1,70 | 3,83 | 3,32 |
| CCR | CLSP357 | 78 | Homme | IV | | 9,37 | 76,31 | 4,52 |
| CCR | CLSP365 | 64 | Homme | IV | | 29,50 | 1135,20 | 6,41 |
| CCR | CLSP366 | 45 | Homme | IV | | 2,63 | 52,40 | 4,62 |
| CCR | GHBD022 | 56 | Homme | IV | | 1369,50 | 21,58 | 20,22 |
| CCR | GHBD071 | 61 | Homme | IV | | 38,02 | 41,09 | 34,61 |
| CCR | GHBD093 | 78 | Homme | IV | | 0,00 | 6,91 | 4,44 |
| CCR | CLSP197 | 68 | Femme | NA | | 0,59 | 10,12 | 7,23 |
| | Seuil (concentration max des ColoNeg) | | | | | 5,81 | 40,93 | 11,06 |
| | Tumeur in situ Tis T0 : Nombre de patients supérieurs au seuil | | | | | 0 | 0 | 5 |

| | LFABP Nombre patients détectés | ACE Nombre patients détectés | CA 19-9 Nombre patients détectés | Nombre total | | | | |
|---|---|---|---|---|---|---|---|---|
| Tis T0 | 5 | 0 | 0 | n= 8 | | | | |
| CCR+ stade I | 3 | 1 | 0 | n=14 | | | | |
| CCR+ stade II | 13 | 8 | 1 | n=49 | | | | |
| CCR+ stade III | 10 | 9 | 3 | n=27 | | | | |
| CCR+ stade IV | 5 | 8 | 8 | n=14 | | | | |

| | | | | | | LFABP | ACE | CA19-9 |
|---|---|---|---|---|---|---|---|---|
| sensibilité Tis T0 / ColoNeg en pourcentage | | | | | | 63 | 0 | 0 |
| sensibilité CCR stade I / ColoNeg | | | | | | 21 | 7 | 0 |
| sensibilité CCR stade II / ColoNeg | | | | | | 27 | 16 | 2 |
| sensibilité CCR stade III / ColoNeg | | | | | | 37 | 33 | 11 |
| sensibilité CCR stade IV / ColoNeg | | | | | | 36 | 57 | 57 |

Selon la Figure 2, on voit que la LFABP permet de détecter 5 patients sur 8 avec une tumeur in situ Tis (TO), sans détecter de patient contrôle coloscopie négative (seuil de spécificité fixé à 100%, avec un seuil de détection pour la LFABP à 11,06 ng/ml). Dans le même temps on a une bonne détection des cancers colorectaux invasifs de stades I à IV par la LFABP.

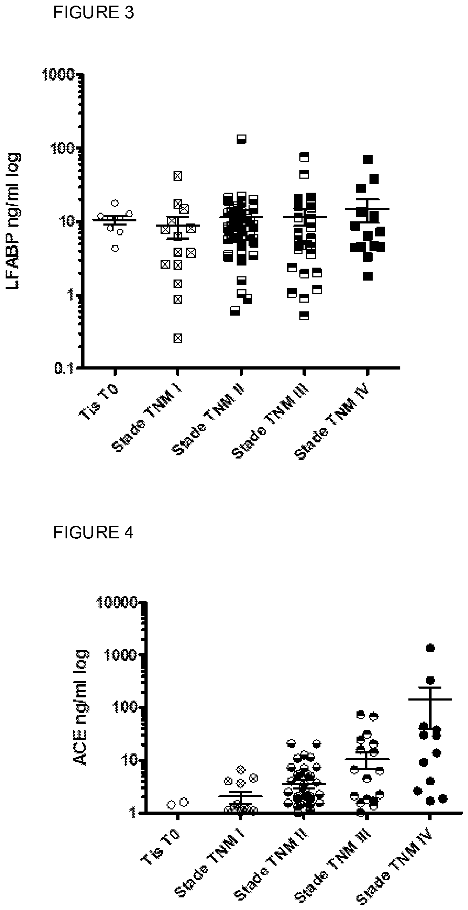

La détection des Tis (TO) par rapport aux coloscopies négatives est statistiquement significative (p=0,0002) avec un t-test de Mann-Whitney. Comme l'est la détection des cancers colorectaux invasifs de stade I à IV, illustrée sur la Figure 3.

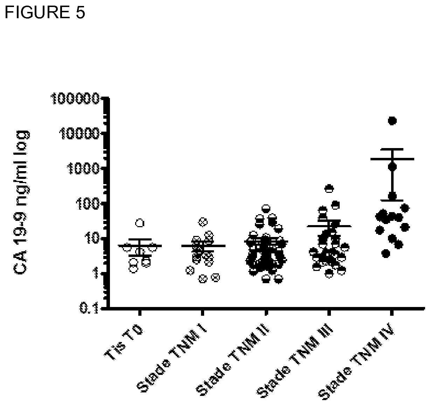

Comme l'illustrent les Figures 4 et 5, comparativement, avec une spécificité fixée à 100% avec le groupe contrôle coloscopies négatives, on ne détecte aucun patient atteint de Tis (TO) avec le marqueur antigène carcino-embryonnaire (ACE). Et aucun patient atteint d'adénome, ni de patient atteint de Tis (TO), ni de patient porteur d'un CCR invasif de stade TNM I avec le marqueur CA19-9, sur la cohorte testée.

L'ACE et le marqueur CA 19-9 sont des marqueurs tumoraux et qu'ils détectent les stades avancés mieux que les stades précoces, le relargage dans le sang du marqueur étant proportionnel à la taille de la tumeur et à son agressivité.

Par contre, la demanderesse a mis en évidence de façon surprenante que la LFABP détecte aussi bien les lésions précancéreuses (adénomes) et les tumeurs in situ TO que les stades précoces et les stades avancés de cancer colorectal invasif, et permet ainsi d'améliorer les chances de survie des patients.

### Exemple 5 : Détection de la LFABP par la technique LC-MRM-MS et MRM cube

### 1) Technique MRM

### 1.1) Méthodologie

Le principe des dosages en utilisant la technologie de dosage Multiple Reaction Monitoring MRM repose sur l'association entre la chromatographie liquide et un spectromètre de masse de type hybride triple quadrupole/trappe ionique (5500Q trap d'AB Sciex) fonctionnant en mode MRM (Q1q2Q3). Dans le principe, pour chaque protéine d'intérêt on choisit les peptides trypsiques d'intérêt. Les transitions sont prédites par exemple à l'aide des logiciels Midas™ puis MRM pilot™ d'AB Sciex.

On quantifie le marqueur LFABP par le biais de la quantification d'un ou plusieurs de ses peptides. Ces peptides sont choisis en fonction de leurs caractéristiques physico-chimique, et de leur capacité à être détecté dans le spectromètre de masse. Les peptides sélectionnés sont isolés dans le premier quadrupole Q1, pour être fragmentés dans la cellule de collision du spectromètre de masse (q2). A partir des ions fils générés, nous avons choisi les plus représentatifs et qui permettaient la meilleure spécificité, et l'intensité de ces ions fils a été monitorée après sélection dans le troisième quadrupole Q3. Le couple de masse ion parent/ion fils est appelé transition, et c'est l'association de la transition au temps de rétention chromatographique qui lui correspond qui définit la spécificité de l'essai par rapport à la protéine cible. Pour avoir un dosage quantitatif, il faut ajouter un standard interne (par exemple un peptide identique « lourd » qui va servir d'étalon, en quantité connue, qui est ajouté à l'échantillon et est élué au même temps chromatographique et qui permet la quantification relative. Les peptides ont été sélectionnés en fonction de leur sensibilité (meilleure réponse dans le spectromètre de masse) et de leur spécificité dans la matrice

Des peptides de séquences identiques aux peptides cibles sélectionnés ont été synthétisés, avec de la lysine ou de l'arginine marquées (C13 et N15) : +8 Dalton pour la lysine, +10 Da pour l'arginine, afin de pouvoir réaliser des dosages quantitatifs. En effets, ces peptides lourds ont les mêmes propriétés physico-chimiques que les peptides cibles, et sont élués aux mêmes temps de rétention chromatographiques.

Afin de pouvoir baisser la limite de détection à quelques ng/ml, un procédé amélioré de MRM-MS a été mis en oeuvre. Les étapes successives de ce procédé sont: 1) digestion trypsique, 2) fractionnement SPE (solid-phase extraction) des peptides, 3) chromatographie liquide (LC) couplée à la MRM-MS.

La mise au point a été réalisée sur des échantillons surchargés (spike) en ajoutant les peptides synthétiques.

### Digestion enzymatique :

Les échantillons de sérums sont dénaturés dans une solution d'urée 6M tamponnée par 10 mM de Tris pH 8 et contenant 30 mM de dithiothreitol, pendant 40 minutes à 40°C, puis alkylés par de l'iodoacétamide 50 mM, à température ambiante, pendant 40 minutes, à l'obscurité. Ils sont dilués 6 fois dans l'eau, puis la digestion trypsique est réalisée à 37°C, sur la nuit, en utilisant un ratio enzyme substrat de 1:30 (Promega). La digestion est arrêtée par ajout d'acide formique à une concentration finale de 0,5%. Les échantillons digérés sont dessalés par extraction sur phase solide (SPE, solid phase extraction) en utilisant les cartouches en phase inverse Oasis HLB 3cc (60 mg) (Waters). Après application de l'échantillon, les cartouches sont lavées par 1 ml d'acide formique à 0,1%, puis l'élution a été réalisée par un mélange méthanol/eau (80/20 v/v) contenant 0,1% d'acide formique. Les éluats sont séchés sous vide.

### Fractionnement SPE :

Les échantillons secs sont repris dans 1 ml de tampon acétate et chargés sur les cartouches mixtes (hydrophobe et échange de cation) Oasis MCX (mixed cation exchange) 60 mg (Waters) équilibrées au préalable en tampon acétate et méthanol. Les cartouches sont lavées par 1 ml de tampon acétate et 1 ml de méthanol. Les peptides d'intérêt (Tableau 8) sont élués par 1 ml d'un mélange méthanol/tampon acétate (50/50 v/v)). Le pH du tampon acétate est choisi en fonction du point isoélectrique du peptide d'intérêt. Les éluats sont séchés sous vide, dissous dans 200 µl d'une solution d'acétonitrile/eau (3/97 v/v) contenant 0,1% d'acide formique. Un aliquot de 50 µl a été injecté dans la LC couplée à un système MS-MS.

### Chromatoaraphie liquide et spectrométrie de masse :

L'analyse LC-MS a été effectuée sur un système chromatographique haute pression (HPLC) de type HP 1100 series avec pompe binaire et injecteur (Agilent Technologies) couplé à un spectromètre de masse, AB Sciex 400 QTrap (MS hybride triple quadripôle - trappe ionique) (MDS Sciex) pour une meilleure sensibilité. La séparation LC a été effectuée sur une colonne C18 Symmetry (Waters), à un débit d'élution de 300 pl/min. (Eluent A = 0,1% acide formique dans l'eau, éluent B = 0,1% acide formique dans l'acétonitrile, gradient linéaire de 5%B à 50%B en 25 min, puis de 50%B à 100%B en 3 min). L'analyse MS est réalisée en mode d'ionisation positive à une tension de 5500 V appliquée à l'aiguille permettant l'ionisation dans la source. Le contrôle de l'instrument et l'acquisition des données sont réalisés avec le logiciel Analyst 1.4.1. Les débits du gaz de nébulisation (air) et du gaz rideau (azote) sont de 30 et 20 psi, respectivement. La source ionique Turbo VTM est réglée à 400°C, le flux d'azote auxiliaire à 40 psi. L'énergie de collision (CE), la tension d'orifice (DP, declustering potential) et la tension à la sortie de la cellule de collision (CXP, collision cell exit potential) sont optimisées pour chacune des transitions MRM sélectionnées.

Tous les ions parents di- ou tri- chargés des peptides tryptiques théoriques dans un intervalle de masse allant de 800 à 3000 Da et tous les ions fragments possibles de type y ou b. Pour chaque protéine, chaque transition possible a été testée afin de déterminer les transitions les plus sensibles et les plus spécifiques. Le résultat de cette sélection est récapitulé dans le Tableau 8. La séparation chromatographique MCX a été réalisée à différents pH, le pH choisi pour la suite des expériences étant le pH qui permet d'obtenir l'aire du pic la plus élevée.

La liste des transitions MRM théoriques des séquences SEQ ID NO : 5 à 7 pour le dosage du marqueur LFABP ont été générées en utilisant le logiciel MIDAS (MRM-initiated Détection and Sequencing). Cette liste comprend 3 peptides de la protéine LFABP, et pour chaque peptide, plusieurs transitions sont suivies :

**Tableau 8**

| Peptides et transitions utilisés pour le dosage de la LFABP | | | | | | | | | | |
|---|---|---|---|---|---|---|---|---|---|---|
| *LFABP* | | | | | | | | | | |
| Séquences (SEQ ID N°) | pl | TR | Q1 (peptide léger naturel) | Q3 (peptide léger naturel) | Q1 (peptide lourd standard interne) | Q3 (peptide lourd standard interne) | DP | EP | CE | CXP |
| AIGLPEELIQK (SEQ ID N°5) | 4,31 | 16,75 | 605,9 | 630,6 | 609,9 | 638,6 | 120 | 2,2 | 35 | 34,5 |
| | | | | 759,7 | | 767,7 | 120 | 2,2 | 34 | 30 |
| | | | | 856,2 | | 864,2 | 120 | 2,2 | 20 | 36 |
| GVSEIVQNGK (SEQ ID N°6) | 6,99 | 9,4 | 515,8 | 545,3 | 519,8 | 553,3 | 108 | 2,2 | 19,8 | 31 |
| | | | | 874,5 | | 882,5 | 108 | 2,2 | 23,3 | 31 |
| | | | | 658,4 | | 666,4 | 108 | 2,2 | 23,3 | 31 |
| TWQLEGDNK (SEQ ID N°7) | 4,11 | 9,65 | 551,8 | 675,3 | 555,8 | 683,3 | 88 | 4,2 | 25,7 | 16 |
| | | | | 803,4 | | 811,4 | 88 | 4,2 | 18,9 | 17,5 |
| | | | | 902,4 | | 910,4 | 88 | 4,2 | 16 | 21 |

De plus, en utilisant un standard interne, par exemple un peptide lourd de type AQUA (Sigma) ou encore une protéine recombinante lourde qui serviront d'étalon de dosage, ou toute autre méthode de standardisation connue de l'homme du métier, il est possible de quantifier de manière absolue le marqueur tumoral d'intérêt dans un milieu biologique complexe.

Dans un mode préféré de l'invention, on suivra la transitions 605,9/856,5 du peptide de SEQ ID NO : 5, et sa transition du peptide lourd correspondant. On établira le ratio des aires du pic chromatographique de la transition naturelle divisée par l'aire de la transition lourde pour obtenir une dose par transition pour chaque échantillon, puis on summerisera entre elles ou non les doses obtenues pour les différentes transitions, par exemple par la méthode médian polish selon la procédure de Tukey.

### 2) Détection de la LFABP par la technique MRM cubed

### 2.1) Méthodologie

Il s'agit d'une nouvelle méthode développée par les auteurs de l'invention, qui permet d'augmenter le ratio signal/bruit et la spécificité analytique d'un dosage. Dans un fluide complexe comme le sérum, l'augmentation de la spécificité analytique permet une augmentation de la sensibilité observée. Cette méthode a un temps de mise au point plus long, et elle implique également un temps d'acquisition plus long pour chaque échantillon.

Avec cette méthode, la LFABP a été quantifiée, par le biais du peptide de SEQ ID NO : 5.

La méthode est développée sur un spectromètre de masse de type hybride triple quadrupole/trappe ionique (5500Q trap d'AB Sciex) et consiste à : i) sélection de l'ion peptidique protéotypique en Q1, ii) activation CID de l'ion sélectionné en Q2, iii) capture d'un des ions fragments les plus intense en Q3, ce qui définit la transition MRM optimale, iv) activation de cet ion fils pour générer des fragments de deuxième génération, v) scan sélectif des masses générées de ces ions de deuxième génération. Après acquisition, un chromatogramme MRM3 est reconstruit après l'extraction de un ou de plusieurs ions produits de deuxième génération pour quantification.

Cette méthodologie très spécifique permet de limiter le pré-traitement d'échantillon. Dans cet exemple, le seul pré-traitement d'échantillon a été une extraction des peptides en phase solide sur une colonne d'échanges cationiques. La plateforme utilise des colonnes chromatographiques conventionnelles de 2,1 mm.

### 2.2) Corrélation entre les différentes méthodes de dosage

Pour s'assurer de la robustesse de nos méthodes de dosage, et de la validité clinique du marqueur LFABP nous avons regardé :
- les coefficients de variation des dosages, leur répétabilité et reproductibilité
- la consistance des dosages en répétant à plusieurs mois d'intervalle les mêmes dosages sur plusieurs échantillons : le coefficient de raccordement pour les dosages Vidas® de la LFABP était de 99%, la corrélation de raccordement pour le test MRM (de SEQ ID NO : 5, 605,9/856,5) LFABP de 98%.
- la corrélation entre les différents dosages. La mesure de la corrélation a été réalisée à la fois par un test de régression linéaire et un test de corrélation de Spearman.

On observe une bonne corrélation entre deux peptides de la protéine LFABP, réalisée par un test sur 182 patients : de SEQ ID NO : 7 et de SEQ ID NO : 5. Ces deux dosages MRM indépendants dosent donc bien la même molécule. On observe aussi une bonne corrélation entre le dosage MRM du peptide de SEQ ID NO : 5 et le dosage ELISA 2C9G6/5A8H2

Comme montré dans le tableau 9 ci-après, la corrélation entre les tests ELISA Vidas® et les tests MRM et MRM3 est bonne (r² supérieur à 0.90 dans tous les cas)

**Tableau 9**

| Corrélation entre les différentes métthodes de dosage évaluée sur 8 Tis, 104 CCR invasifs, 70 patients donneurs de sang et 31 ColoNeg | | | | | |
|---|---|---|---|---|---|
| | MRM SEQ ID NO: 5 856,5 | MRM3 | Vidas® 2C9G6 / 5A8H2 | Vidas® 5A8H2 / 3D6G1 | ELISA Hycult biotech |
| MRM de SEQ ID NO : 5 605,9/856,5 | 1 | 0.94 | 0.90 | 0.92 | 0.80 |
| MRM3 | 0.94 | 1 | 0.90 | 0.90 | 0.77 |
| VIDAS® 2C9G6 / 5A8H2 | 0.90 | 0.90 | 1 | 0.96 | 0.73 |
| Vidas® 5A8H2 / 3D6G1 | 0.92 | 0.90 | 0.96 | 1 | 0.81 |
| ELISA commercial | 0.80 | 0.77 | 0.73 | 0.81 | 1 |

## Revendications

1. Procédé de détection d'une lésion colorectale non cancéreuse susceptible d'évoluer en cancer colorectal invasif, chez un patient n'ayant pas de cancer colorectal invasif,
par la détermination de la présence de la Liver Fatty Acid-Binding Protein (LFABP), dans un échantillon biologique du patient, ledit échantillon étant choisi parmi le sang total, le sérum et le plasma, et
par la mise en évidence d'une variation de l'expression de la LFABP par comparaison à une population de référence dite contrôle.

2. Procédé selon la revendication 1, **caractérisé en ce que** la lésion colorectale est un adénome colorectal,

3. Procédé selon la revendication 1, **caractérisé en ce que** la lésion est un Tis TO.

4. Procédé selon l'une quelconque des revendications précédentes, **caractérisé en ce qu'**on détecte la LFABP et/ou au moins un traceur représentatif de la LFABP et choisi parmi des peptides, des acides nucléiques, des anticorps et/ou on mesure au moins une activité biologique, par exemple enzymatique spécifique de la LFABP, dans ledit échantillon biologique.

5. Procédé selonl'une quelconque des revendications précédentes, **caractérisé en ce qu'**on met en évidence une augmentation de l'expression de la LFABP.

6. Procédé selon l'une quelconque des revendications précédentes, **caractérisé en ce qu'**on détermine la présence de la LFABP par une technique de spectrométrie de masse.

7. Procédé selon la revendication 6, **caractérisé en ce qu'**on détermine la présence de la LFABP par la technique LC-MRM-MS qui combine la chromatographie liquide et la spectrométrie de masse en mode MRM (Mutiple Reaction Monitoring).

8. Procédé selon l'une quelconque des revendications 1 à 5, **caractérisé en ce qu'**on détermine la présence de la LFABP par une technique immunologique.

9. Procédé selon l'une quelconque des revendications précédentes, **caractérisé en ce qu'**on détermine la présence de la LFABP par une méthode combinant technique de spectrométrie de masse et technique immunologique.

10. Procédé selon la revendication 8 ou 9, **caractérisé en ce qu'**on détermine la présence de la LFABP avec des anticorps reconnaissant spécifiquement un peptide de séquence choisie parmi SEQ ID NO : 1, SEQ ID NO : 2, SEQ ID NO : 3, et SEQ ID NO : 4.

11. Procédé selon l'une quelconque des revendications 1 à 5, **caractérisé en ce qu'**on détermine la présence de la LFABP par une technique enzymatique.

12. Procédé selon l'une quelconque des revendications 6 à 11, **caractérisé en ce qu'**on détecte un peptide ou un mélange de peptides choisis parmi SEQ ID NO : 1, SEQ ID NO : 2, SEQ ID NO : 3, SEQ ID NO : 4, SEQ ID NO : 5, SEQ ID NO 6 et SEQ ID NO : 7.

13. Procédé de détection, in vitro, d'une susceptibilité à un cancer colorectal chez un patient, **caractérisé en ce qu'**on met en oeuvre un procédé selon l'une quelconque des revendications précédentes.

## Patentansprüche

1. Verfahren zur Erkennung einer nicht krebsartigen kolorektalen Läsion, die sich zu einem invasiven kolorektalen Krebs entwickeln könnte, bei einem Patienten, der keinen invasiven kolorektalen Krebs hat,
durch die Bestimmung der Anwesenheit des Liver Fatty Acid-Binding Protein (LFABP) in einer biologischen Probe des Patienten, wobei die Probe aus dem Vollblut, dem Serum und dem Plasma ausgewählt ist, und
durch den Nachweis einer Variation der Expression des LFABP durch Vergleichen mit einer als Kontrolle bezeichneten Referenzpopulation.

2. Verfahren nach Anspruch 1, **dadurch gekennzeichnet, dass** die kolorektale Läsion ein kolorektales Adenom ist.

3. Verfahren nach Anspruch 1, **dadurch gekennzeichnet, dass** die Läsion ein Tis TO ist.

4. Verfahren nach einem der vorangehenden Ansprüche, **dadurch gekennzeichnet, dass** das LFABP und/oder mindestens ein repräsentativer Tracer des LFABP und ausgewählt aus Peptiden, Nukleinsäuren, Antikörpern erkannt und/oder mindestens eine biologische Aktivität, beispielsweise enzymatische, spezifisch für das LFABP, in der biologischen Probe gemessen wird.

5. Verfahren nach einem der vorangehenden Ansprüche, **dadurch gekennzeichnet, dass** der Nachweis einer Erhöhung der Expression des LFABP erfolgt.

6. Verfahren nach einem der vorangehenden Ansprüche, **dadurch gekennzeichnet, dass** die Anwesenheit des LFABP durch eine Massenspektrometrietechnik bestimmt wird.

7. Verfahren nach Anspruch 6, **dadurch gekennzeichnet, dass** die Anwesenheit des LFABP durch die Technik LC-MRM-MS bestimmt wird, welche die Flüssigchromatographie und die Massenspektrometrie im MRM-Modus (Mutiple Reaction Monitoring) kombiniert.

8. Verfahren nach einem der Ansprüche 1 bis 5, **dadurch gekennzeichnet, dass** die Anwesenheit des LFABP durch eine immunologische Technik bestimmt wird.

9. Verfahren nach einem der vorangehenden Ansprüche, **dadurch gekennzeichnet, dass** die Anwesenheit des LFABP durch eine Methode bestimmt wird, welche Massenspektrometrietechnik und immunologische Technik kombiniert.

10. Verfahren nach Anspruch 8 oder 9, **dadurch gekennzeichnet, dass** die Anwesenheit des LFABP mit Antikörpern bestimmt wird, welche speziell ein Peptid der Sequenz erkennen, die aus der SEQ ID NO : 1, SEQ ID NO : 2, SEQ ID NO : 3 und SEQ ID NO : 4 ausgewählt ist.

11. Verfahren nach einem der Ansprüche 1 bis 5, **dadurch gekennzeichnet, dass** die Anwesenheit des LFABP durch eine enzymatische Technik bestimmt wird.

12. Verfahren nach einem der Ansprüche 6 bis 11, **dadurch gekennzeichnet, dass** ein Peptid oder ein Peptidgemisch erkannt wird, ausgewählt aus SEQ ID NO : 1 , SEQ ID NO : 2, SEQ ID NO : 3, SEQ ID NO : 4, SEQ ID NO : 5, SEQ ID NO : 6 und SEQ ID NO : 7.

13. Verfahren zur Erkennung in vitro einer Wahrscheinlichkeit eines kolorektalen Krebses bei einem Patienten, **dadurch gekennzeichnet, dass** ein Verfahren nach einem der vorangehenden Ansprüche durchgeführt wird.

## Claims

1. A method for detecting a non-cancerous colorectal lesion that may develop into invasive colorectal cancer in a patient who does not have invasive colorectal cancer,
by determining the presence of the Liver Fatty Acid-Binding Protein (LFABP) in a biological sample of the patient, said sample being selected from the whole blood, the serum and the plasma, and
by highlighting a variation in the expression of LFABP compared to a reference population called control population.

2. The method according to claim 1, **characterized in that** the colorectal lesion is a colorectal adenoma.

3. The method according to claim 1, **characterized in that** the lesion is a Tis T0.

4. The method according to any one of the preceding claims, **characterized in that** the LFABP and/or at least one tracer representative of the LFABP and selected from peptides, nucleic acids, antibodies is/are detected and/or at least one biological activity, for example enzymatic activity specific to the LFABP, in said biological sample, is measured.

5. The method according to any one of the preceding claims, **characterized in that** an increase in the expression of LFABP is highlighted.

6. The method according to any one of the preceding claims, **characterized in that** the presence of LFABP is determined by a mass spectrometry technique.

7. The method according to claim 6, **characterized in that** the presence of LFABP is determined by the LC-MRM-MS technique which combines liquid chromatography and mass spectrometry in MRM (Multiple Reaction Monitoring) mode.

8. The method according to any one of claims 1 to 5, **characterized in that** the presence of LFABP is determined by an immunological technique.

9. The method according to any one of the preceding claims, **characterized in that** the presence of LFABP is determined by a method combining a mass spectrometry technique and an immunological technique.

10. The method according to claim 8 or 9, **characterized in that** the presence of LFABP is determined with antibodies specifically recognizing a peptide of a sequence selected from SEQ ID NO: 1, SEQ ID NO: 2, SEQ ID NO: 3, and SEQ ID NO: 4.

11. The method according to any one of claims 1 to 5, **characterized in that** the presence of LFABP is determined by an enzymatic technique.

12. The method according to any one of claims 6 to 11, **characterized in that** a peptide or a mixture of peptides selected from SEQ ID NO: 1, SEQ ID NO: 2, SEQ ID NO: 3, SEQ ID NO: 4, SEQ ID NO: 5, SEQ ID NO 6 and SEQ ID NO: 7 is detected.

13. The method for detecting, in vitro, a susceptibility to colorectal cancer in a patient, **characterized in that** a method is implemented according to any one of the preceding claims.
